(19) Europäisches Patentamt
European Patent Office
Office européen des brevets

(11) EP 1 490 694 B1

(12) EUROPEAN PATENT SPECIFICATION

(45) Date of publication and mention of the grant of the patent:
**09.03.2011 Bulletin 2011/10**

(21) Application number: **03722373.2**

(22) Date of filing: **01.04.2003**

(51) Int Cl.:
***G01N 33/68*** *(2006.01)* ***C12Q 1/68*** *(2006.01)*

(86) International application number:
**PCT/EP2003/003364**

(87) International publication number:
**WO 2003/083482 (09.10.2003 Gazette 2003/41)**

# (54) CAMP-REGULATED PHOSPHORPROTEIN FOR DIAGNOSTIC AND THERAPEUTIC USE IN NEURODEGENERATIVE DISEASES

CAMP-REGULIERTES PHOSPHOPROTEIN FÜR DIE DIAGNOSE UND BEHANDLUNG VON NEURODEGENERATIVEN KRANKHEITEN

PHOSPHOPROTEINE A REGULATION AMPC POUR DIAGNOSTIC ET SOINS DANS DES MALADIES NEURODEGENERATIVES

(84) Designated Contracting States:
**AT BE BG CH CY CZ DE DK EE ES FI FR GB GR HU IE IT LI LU MC NL PT RO SE SI SK TR**

(30) Priority: **02.04.2002 EP 02007522**
**02.04.2002 US 368970 P**

(43) Date of publication of application:
**29.12.2004 Bulletin 2004/53**

(73) Proprietor: **EVOTEC Neurosciences GmbH**
**22525 Hamburg (DE)**

(72) Inventors:
- **HIPFEL, Rainer**
  **69126 Heidelberg (DE)**
- **HANES, Jozef**
  **84107 Bratislava (SK)**
- **VON DER KAMMER, Heinz**
  **22607 Hamburg (DE)**
- **POHLNER, Johannes**
  **22175 Hamburg (DE)**

(74) Representative: **von Kreisler Selting Werner**
**Deichmannhaus am Dom**
**Bahnhofsvorplatz 1**
**50667 Köln (DE)**

(56) References cited:
**US-A- 5 777 195**

- **DATABASE EBI [Online] Database accession no. AL133109 XP002218529**
- **BRENE STEFAN ET AL: "Expression of mRNAs encoding ARPP-16/19, ARPP-21, and DARPP-32 in human brain tissue." JOURNAL OF NEUROSCIENCE, vol. 14, no. 3 PART 1, 1994, pages 985-998, XP001107003 ISSN: 0270-6474**
- **KISIELOW, J. ET AL: "TARPP, a novel protein that accompanies TCR gene rearrangement and thymocyte education" EUR. J. IMMUNOL., no. 31, 2001, pages 1141-1149, XP001107040**
- **KIM, S. H. ET AL: "Decreased levels of ARPP-19 and PKA in brains of Down syndrome and Alzheimer's disease" J NEURAL TRANSM, no. 61, 2001, pages 263-272, XP001117653**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 490 694 B1

**Description**

The present invention relates to methods of diagnosing, prognosticating and monitoring the progression of neurodegenerative diseases in a subject. Furthermore, methods of therapy control and screening for modulating agents of neurodegenerative diseases are provided. The invention also discloses pharmaceutical compositions, kits, and recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). Two types of plaques, diffuse plaques and neuritic plaques, can be detected in the brain of AD patients, the latter ones being the classical, most prevalent type. They are primarily found in the cerebral cortex and hippocampus. The neuritic plaques have a diameter of 50μm to 200μm and are composed of insoluble fibrillar amyloids, fragments of dead neurons, of microglia and astrocytes, and other components such as neurotransmitters, apolipoprotein E, glycosaminoglycans, α1-antichymotrypsin and others. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. Along the formation of NFTs, a loss of neurons can be observed. It is discussed that said neuron loss may be due to a damaged microtubule-associated transport system (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376). AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92). The age of onset of AD may vary within a range of 50 years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). The polymorphic plasmaprotein ApoE plays a role in the intercellular cholesterol and phospholipid transport by binding low-density lipoprotein receptors, and it seems to play a role in neurite growth and regeneration. Efforts to detect further susceptibility genes and disease-linked polymorphisms, lead to the assumption that specific regions and genes on human chromosomes 10 and 12 may be associated with late-onset AD (Myers et al., Science 2000, 290: 2304-5; Bertram et al., Science 2000, 290: 2303; Scott et al., Am J Hum Genet 2000, 66: 922-32).

Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The mutations found to date account for only half of the familial AD cases, which is less than 2% of all AD patients. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

A group of cAMP-regulated phosphoproteins (ARPPs) has been shown to function as so called intracellular third-messengers in the mammalian central nervous system. Receptor-mediated phosphorylation and dephosphorylation of ARPPs constitute important pathways for the regulation of neuronal functions in response to levels of the important second messenger cAMP and activity of the cAMP-dependent protein kinase, PKA (for recent review, Greengard, Science 2001, 294: 1024-1030). To date, the best characterized ARPPs are DARPP-32 (dopamine and cAMP regulated phosphoprotein of 32 kDa molecular weight), ARPP-16/19 (cAMP regulated phosphoprotein of 16/19 kDa molecular weight), and ARPP-21 (cAMP regulated phosphoprotein of 21 kDa molecular weight), all of which are encoded by separate genes in the human genome. DARPP-32 is encoded on chromosome 17, ARPP-16/19 on chromosome 15, and the ARPP-21 locus is found on chromosome 3 of the human genome. DARPP-32, ARPP-16/19, and ARPP-21 are non-homologous proteins but may have similar or even overlapping functions based on their tissue expression pattern within the human post-mortem brain. Using in situ hybridization techniques, transcripts for all three ARPPs can be detected in brain regions that receive a rich dopamine innervation from the mesencephalon, i.e. the caudate nucleus, putamen, nucleus accumbens, and the amygdaloid complex. ARPP-16/19, in addition, shows a strong mRNA hybridization signal in the neocortex, whereas DARPP-32 and ARPP-21 showed low levels of signal intensity only (Brene et al., J Neurosci 1994, 14: 985-998). The distribution of ARPP mRNAs overlaps to a large extent with the distribution of the dopamine D1 receptor which thus may regulate the phophorylation status of ARPPs via adenylate cyclase/cAMP and PKA. In fact, the phosphorylation status of DARPP-32 is at the crossroads of multiple complex signaling pathways involving PKA (signaling by receptors for dopamine, opiate, adenosine, serotonin, vasoactive intestinal peptide), the protein phosphatase PP-2B/calcineurin (signaling by receptors for dopamine, gamma-aminobutyric acid, glutamate), and the protein phosphatase PP-1 which controls the state of phosphorylation and activity of numerous physiologically important substrates including neurotransmitter receptors, voltage-gated ion channels, ion pumps, and transcription factors (Greengard, Science 2001, 294: 1024-1030).

A function of ARPP-21 is largely unknown. Human ARPP-21 consists of 89 amino acids and is phophorylated by PKA on serin-56 (Brene et al., J Neurosci 1994, 14: 985-998). The human ARPP-21 isoform cARPP encoding a polypeptide of 89 amino acids has been described in WO00/34477. Available evidence supports the view that ARPP-21 is a cAMP regulated phosphoprotein highly enriched in the cell bodies and terminals of medium-sized spiny neurons of the basal ganglia with the highest levels of immunoreactivity seen in structures comprising the limbic striatum (Ouimet et al., J Neurosci 1989, 9: 865-875). ARPP-21 may therefore play a role as an intracellular third messenger in mediating some of the effects of dopamine, vasoactive intestinal polypeptide, and/or other neurotransmitters acting via cAMP in these dopamine-innervated brain regions (Ouimet et al., J Neurosci 1989, 9: 865-875; Hemmings and Greengard, J Neurosci 1989, 9: 851-864). In fact, the dopamine D1 agonist SKF38393 was shown to increase the state of phosphorylation of ARPP-21 in tissue slices of the substantia nigra of rat brain (Tsou et al., J Neurochem 1993, 60: 1043-1046). Using mouse striatal slices these results were recently corroborated and extended to show that agonists of dopamine D2 receptors cause a strong decrease in ARPP-21 phosphorylation (Caporaso et al., Neuropharmacology 2000, 39: 1637-1644). The likely effector of the dopamine D2 receptor signal is the protein phophatase PP-2A. Several neurological and psychiatric diseases are associated with abnormalities in the dopamine signaling pathways, among them Parkinson's disease, schizophrenia, attention deficit hyperactivity disorder, and drug abuse. A dysregulation of DARPP-32 function has been postulated to be causally related to the above disorders and, therefore, DARPP-32 can be considered a potential therapeutic target for said diseases (WO 99/20273; US 5777195). A recent study correlates levels of the cAMP regulated phosphoprotein ARPP-19 mRNA and protein in brain tissue from patients suffering from Down syndrome or from Alzheimer's disease (Kim et al., J Neural Transm Suppl 2001, 61: 263-272). Kim and coworkers find normal levels of ARPP-19 mRNA in the temporal lobe and a reduced level of ARPP-19 protein in the cerebellum of AD brain tissue when compared to normal brain.

The present disclosure provides a defined pathophysiological implication and diagnostic and therapeutic utility for a novel and hitherto undescribed human isoform of the cAMP regulated phosphoprotein ARPP-21, herein designated as human TARPP (hTARPP), on the basis of differential expression of hTARPP mRNA in post-mortem brains of patients suffering from Alzheimer's disease in comparison to age-matched healthy individuals. In the mouse, a homologous ARPP-21 splice-variant, called TARPP, encodes a ca. 100 kDa protein that accompanies T cell receptor gene rearrangement and thymocyte education (Kisielow et al., Eur J Immunol 2001, 31: 1141-1149). The name "TARPP" was coined to reflect the thymocyte-specific protein expression in mice. However, murine TARPP mRNA and protein can also be detected in the brain, whereas no mRNA or protein is found in heart, lung, liver, lymph nodes, and spleen. A function for murine TARPP in the brain has not been described.

To date, no experiments have been described that demonstrate a link between the dysregulation of ARPP-21 gene expression and neurodegenerative disorders. Particularly the disclosure in the present invention of the novel human TARPP (hTARPP) isoform, and the identification of a link of this isoform with neurodegenerative diseases, offer new ways, inter alia, for the diagnosis and treatment of neurodegenerative disorders, in particular Alzheimer's disease.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene. The terms "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared. The term "variant" as used herein refers to any polypeptide and protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of the polypeptides and proteins herein. "Variants" shall also comprise a sequence that has at least about 80% sequence identity, more preferably at least about 90% sequence identity, and most preferably at least about 95% sequence identity with the amino acid sequences of SEQ ID NO. 1. Derivatives, variants and fragments of hTARPP may include, but are not limited to functional consensus binding motifs for PLC$\gamma$ and Grb2, as well as an R3H domain or other functional modules within the polypeptide sequence of hTARPP. Variants of a protein molecule shown in SEQ ID NO. 1 include, for example, proteins with conservative amino acid substitutions in highly conservative regions. For example, isoleucine, valine and leucine can each be substituted for one another. Aspartate and glutamate can be substituted for each other. Glutamine and asparagine can be substituted for each other. Serine and threonine can be substituted for each other. Amino acid substitutions in less conservative regions include, for example, isoleucine, valine and leucine, which can each be substituted for one another. Aspartate and glutamate can be substituted for each other. Glutamine and asparagine can be subsituted for each other. Serine and threonine can be substituted for each other. Glycine and alanine can be substituted for each other. Alanine and valine can be substituted for each other. Methionine can be substituted for each of leucine, isoleucine or valine, and vice versa. Lysine and arginine can be substituted for each other. One of aspartate and glutamate can be substituted for one of arginine or lysine, and vice versa. Histidine can be substituted for arginine or lysine, and vice versa. Glutamine and glutamate can be substituted for each other. Asparagine and aspartate can be substituted for each other. Other examples of protein modifications include glycosylation and further post-translational modifications. "Proteins and polypeptides" of the present invention include variants, fragments, and chemical derivatives of the protein comprising SEQ ID NO. 1. As used herein, protein and polypeptide refer to a linear series of amino acid residues connected to one another by peptide bonds between the alpha-amino group and caboxy groups of adjactent amino acid residues. Other covalent bonds, such as amide and disulfide bonds, may also be present. They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants.

The term "isolated" as used herein is considered to refer to molecules that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state, and that such molecules can be produced by recombinant and/or synthetic means. Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.

The term 'AD' shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998). Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, age-related macular degeneration, narcolepsy, motor neuron diseases, prion diseases, traumatic nerve injury and repair, and multiple sclerosis.

The invention features an isolated nucleic acid encoding a protein molecule shown in SEQ ID NO. 1. Hereinafter, the protein molecule of SEQ ID NO. 1 is denoted human TARPP (hTARPP). Subject to the protein modules of SEQ ID NO. 1, i.e. putative consensus binding motifs for PLCγ and Grb2, as well as an R3H domain, which is a conserved sequence motif, discussed to be involved in the binding of polynucleotides, DNA, single-stranded DNA, and RNA, human TARPP may function as a cAMP regulated protein, as an intracellular third messenger, and/or as a scaffolding protein. Human TARPP may interact with lipids and other proteins, or it may be implicated in nucleic acid binding, in nerve cell signaling pathways, and in organizing and regulating neuronal function, and thus hTARPP may play a role in neurodegeneration, in cell protection and regeneration processes. Also disclosed one functional variants, derivatives and fragments of hTARPP, which might have a modification of the given primary structure of hTARPP, but whose essential biological function may remain unaffected.

The invention also features an isolated nucleic acid molecule, wherein the nucleic acid molecule is a DNA molecule. Nucleic acid molecules can be DNA molecules, such as genomic DNA molecules or cDNA molecules, or RNA molecules, such as mRNA molecules. In particular, said nucleic acid molecules can be cDNA molecules comprising a nucleotide sequence of SEQ ID NO. 2 or SEQ ID NO. 3.

The invention also features an isolated D N A molecule capable of hybridizing with the complement of the cD N A described in SEQ ID NO. 2 or SEQ ID NO. 3 under stringent conditions. Stringent conditions means that hybridization will be carried out 5°C to 10°C below that temperature at which totally complementary nucleic acids will just hybridize. Optimized stringency conditions for each sequence are established on parameters such as temperature, nucleic acid molecule consistency, salt conditions, and others well known to those of ordinary skill in the art (see Sambrook and Russell, Molecular Cloning: A Laboratory Manual, Cold Spring Harbor Laboratory Press, Cold Spring Harbor, New York, 2000). Examples for stringent conditions include (i) 0.2xSSC (standard saline citrate) and 0.1 % SDS at 60 °C and (ii) 50 % formamide, 4xSSC, 50 mM HEPES, pH 7.0, 10x Denhardt's solution, 100 μg/ml thermally denatured salmon sperm DNA at 42 °C. This shall not exclude even higher stringency conditions as mentioned, nor shall it exclude lower stringency conditions as mentioned.

In another aspect, the invention features a vector comprising a nucleic acid molecule according to one of claims 1 to 4. In preferred embodiments said vector is a virus, a bacteriophage, or a plasmid. Disclosed is a plasmid adapted for expression in a bacterial cell comprising said nucleic acid molecule, encoding a protein molecule shown in SEQ ID NO. 1, or a fragment, or variant, or derivative thereof, and the regulatory elements necessary for expression of said molecule in a bacterial cell.

Further disclosed is a plasmid adapted for expression in a yeast cell which comprises a nucleic acid molecule, encoding a protein molecule shown in SEQ ID NO. 1, or a variant, or fragment, or derivative thereof, and the regulatory elements necessary for expression of said molecule in a yeast cell. Also discloses is a plasmid adapted for expression in a mammalian cell which comprises a nucleic acid molecule, encoding a protein molecule shown in SEQ ID NO. 1, or a fragment, or variant, or derivative thereof, and the regulatory elements necessary for expression of said molecule in a mammalian cell.

In a further aspect, the invention features a cell transformed with a nucleic acid molecule according to one of claims 1 to 4, wherein said cell is in particular a bacterial cell, a yeast cell, a mammalian cell, or an insect cell, provided that the cell is not derived from human embryo. The present invention also discloses cells comprising a DNA molecule capable of hybridizing with the complement of the cDNA described in SEQ ID NO. 2 or SEQ ID NO. 3 under stringent conditions. Disclosed is a bacterial cell comprising a plasmid adapted for expression in a bacterial cell, said plasmid comprising a nucleic acid molecule encoding a protein molecule shown in SEQ ID NO. 1, or a fragment, or a derivative, or a variant thereof, and the regulatory elements necessary for expression of said molecule in the bacterial cell. The invention also discloses a yeast cell comprising a plasmid adapted for expression in a yeast cell, said plasmid comprises a nucleic acid molecule encoding a protein molecule shown in SEQ ID NO. 1, or a fragment, or a derivative, or a variant thereof, and the regulatory elements necessary for expression of said molecule in the yeast cell. Further disclosed is a mammalian cell comprising a plasmid adapted for expression in a mammalian cell, said plasmid comprising a nucleic acid molecule encoding a protein molecule shown in SEQ ID NO. 1, or a variant, or a derivative, or a fragment thereof, and the regulatory elements necessary for expression of said molecule in the mammalian cell.

In one aspect the present invention features a protein molecule shown in SEQ ID NO. 1. Furthermore, the present invention features a protein molecules shown in SEQ ID NO. 1 for use as a diagnostic target for detecting Alzheimer's disease.

The present invention further discloses a protein molecule shown in SEQ ID NO.1, or a fragment, or derivative, or variant thereof, for use as a screening target for reagents or compounds preventing, or treating, or ameliorating a neurodegenerative disease, preferably Alzheimer's disease.

The invention further features an antibody specifically immunoreactive with an immunogen, wherein said immunogen is a protein molecule shown in SEQ ID NO. 1. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunopreoipitation and antibody microarrays. These methods involve the detection of translation products of the human TARPP gene.

The present invention features the use of said antibody for detecting the Alzheimer's disease state of a cell in a sample from a subject, comprising immunocytochemical staining of said cell with said antibody, wherein an altered degree of staining, or an altered staining pattern in said cell compared to a cell representing a known health status indicates the Alzheimer's disease state of said cell. The invention is particularly suited to detect pathological structures in the brain of a subject. It is also especially suited to detect pathological cells of the muscular system, prostate, stomach, testis, ovary, adrenal glands, mammary glands, liver, spleen, lung, trachea or placenta. The pathological state relates to Alzheimer's disease. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

In one aspect, the invention features a method of diagnosing or prognosticating Alzheimer's disease in a subject, or determining whether a subject is at increased risk of developing said disease. The method comprises: determining a level, or an activity, or both said level and said activity of (I) a transcription product of the gene coding for hTARPP, and/or of (ii) a translation product of the gene coding for hTARPP, in a brain sample obtained from said subject and comparing said level, and/or said activity to a reference value representing a known disease or health status, thereby diagnosing or prognosticating said disease in said subject, or determining whether said subject is at increased risk of developing said disease.

The invention also relates to the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and/or variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects.

Whether an amplification results in a specific nucleic acid product or not, and whether a fragment of different length can be obtained or not, may be indicative for Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with Alzheimer's disease. This feature has utility for developing rapid DNA-based diagnostic tests, preferably also in the format of a kit.

Disclosed is a method of monitoring the progression of a neurodegenerative disease in a subject. A level, or an activity, or both said level and said activity, of (i) a transcription product of the gene coding for hTARPP, and/or of (ii) a translation product of the gene coding for hTARPP, and/or of (iii) a fragment, or derivative, or variant of said transcription or translation product in a sample from said subject is determined. Said level and/or said activity is compared to a reference value representing a known disease or health status. Thereby the progression of said neurodegenerative disease in said subject is monitored.

Further disclosed is a method of evaluating a treatment for a neurodegenerative disease, comprising determining a level, or an activity, or both said level and said activity of (i) a transcription product of the gene coding for hTARPP, and/or of (ii) a translation product of the gene coding for hTARPP, and/or of (iii) a fragment, or derivative, or variant of said transcription or translation product in a sample obtained from a subject being treated for said disease. Said level, or said activity, or both said level and said activity are compared to a reference value representing a known disease or health status, thereby evaluating the treatment for said neurodegenerative disease.

The present invention discloses the differential expression and regulation of hTARPP in specific brain regions of Alzheimer's disease patients. Consequently, the gene coding for hTARPP and its corresponding translation products may have a causative role in the regional selective neuronal degeneration typically observed in Alzheimer's disease. Alternatively, hTARPP may confer a neuroprotective function to the remaining surviving nerve cells. Based on these disclosures, the present invention has utility for the diagnostic evaluation and prognosis as well as for the identification of a predisposition to a neurodegenerative disease, in particular Alzheimer's disease. Furthermore, the present invention discloses methods for the diagnostic monitoring of patients undergoing treatment for such a disease.

It is preferred that the sample to be analyzed and determined is selected from the group comprising brain tissue, or other tissues, or other body cells. The sample can also comprise cerebrospinal fluid or other body fluids including saliva, urine, blood, serum plasma, or mucus. Preferably, the methods of diagnosis, prognosis, monitoring the progression or evaluating a treatment for Alzheimer's disease, according to the instant invention, can be practiced ex corpore, and such methods preferably relate to samples, for instance, body fluids or cells, removed, collected, or isolated from a subject or patient.

In further preferred embodiments, said reference value is that of a level, or an activity, or both said level and said activity of (i) a transcription product of the gene coding for hTARPP, and/or of (ii) a translation product of the gene coding for hTARPP, in a sample from a subject not suffering from Alzheimer's disease.

In preferred embodiments, an alteration in the level and/or activity of a transcription product of the gene coding for human TARPP and/or a translation product of the gene coding for human TARPP protein in a sample cell, or tissue, or body fluid in particular cerebrospinal fluid from said subject relative to a reference value representing a known health status indicates a diagnosis, or prognosis, or increased risk of Alzheimer's disease in said subject.

In preferred embodiments, measurement of the level of transcription products of the gene coding for hTARPP is performed in a sample from a subject using a quantitative PCR-analysis with primer combinations to amplify said gene specific sequences from cDNA obtained by reverse transcription of RNA extracted from a sample of a subject. A Northern blot with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of the gene coding for hTARPP, and/or the level of activity of said translation product of the gene coding for hTARPP can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In a preferred embodiment, the level, or the activity, or both said level and said activity of (i) a transcription product of the gene coding for hTARPP, and/or of (ii) a translation product of the gene coding for hTARPP, in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

Disclosed is a kit for diagnosing or prognosticating neurodegenerative diseases, in particular Alzheimer's disease, or determining the propensity or predisposition of a subject to develop a neurodegenerative disease, in particular Alzheimer's disease, said kit comprising:

(a) at least one reagent which is selected from the group consisting of (i) reagents that selectively detect a transcription product of the gene coding for hTARPP, (ii) reagents that selectively detect a translation product of the gene coding for hTARPP; and

(b) instruction for diagnosing, or prognosticating a neurodegenerative disease, in particular Alzheimer's disease, or determining the propensity or predisposition of a subject to develop such a disease by

- detecting a level, or an activity, or both said level and said activity, of said transcription product and/or said translation product of the gene coding for hTARPP, in a sample from said subject; and
- diagnosing or prognosticating a neurodegenerative disease, in particular Alzheimer's disease, or determining the propensity or predisposition of said subject to develop such a disease,

wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status, indicates a diagnosis or prognosis of a neurodegenerative disease, in particular Alzheimer's disease, or an increased propensity or predisposition of developing such a disease. The kit may be particularly useful for the identification of individuals that are at risk of developing a neurodegenerative disease, in particular Alzheimer's disease. Consequently, the kit may serve as a means for targeting identified individuals for early preventive measures or therapeutic intervention prior to disease onset, before irreversible damage in the course of the disease has been inflicted. Furthermore, in preferred embodiments, the kit is useful for monitoring a progression of Alzheimer's disease, in a subject, as well as monitoring success or failure of therapeutic treatment for such a disease of said subject.

The invention discloses a method of treating or preventing a neurodegenerative disease, in particular Alzheimer's disease; in a subject comprising the administration to said subject in a therapeutically or prophylactically effective amount of an agent or agents which directly or indirectly affect a level, or an activity, or both said level and said activity, of (i) the gene coding for hTARPP, and/or (ii) a transcription product of the gene coding for hTARPP, and/or (iii) a translation product of the gene coding for hTARPP, and/or (iv) a fragment, or derivative, or variant of (i) to (iii). Said agent may comprise a small molecule, or it may also comprise a peptide, an oligopeptide, or a polypeptide. Said peptide, oligopeptide, or polypeptide may comprise an amino acid sequence shown in SEQ ID NO. 1, or a fragment, or derivative, or a variant thereof. An agent for treating or preventing a neurodegenerative disease, in particular AD, may also consist of a nucleotide, an oligonucleotide, or a polynucleotide. Said oligonucleotide or polynucleotide may comprise a nucleotide sequence of the gene coding for hTARPP shown in SEQ ID NO. 2 or SEQ ID NO. 3, either in sense orientation or in antisense orientation.

In preferred embodiments, the method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, Acc Chem Res 1993, 26: 274-278 and Mulligan, Science 1993, 260: 926-931) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, Curr Opin Neurobiol 1993, 3: 743-748).

In particular, the invention discloses a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, DN&P 1992, 5: 389-395; Agrawal and Akhtar, Trends Biotechnol 1995, 13: 197-199; Crooke, Biotechnology 1992, 10: 882-6). Apart from hybridization strategies, the application of ribozymes, i.e. RNA molecules that act as enzymes, destroying RNA that carries the message of disease has also been described (see e.g. Barinaga, Science 1993, 262: 1512-1514). In preferred embodiments, the subject to be treated is a human, and therapeutic antisense nucleic acids or derivatives thereof are directed against hTARPP. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, Trends Biotechnol 1992, 10: 281-287). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy. A recently developed method of regulating the intracellular expression of genes by the use of double-stranded RNA, known variously as RNA interference (RNAi), can be another effective approach for nucleic acid therapy (Hannon, Nature 2002, 418: 244-251).

In further preferred embodiments, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection, and liposomal mediated transfection (see Mc Celland and Pardee, Expression Genetics: Accelerated and High-Throughput Methods, Eaton Publishing, Natick, MA, 1999).

In preferred embodiments, said agent for treating and preventing Alzheimer's disease, is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

Methods of treatment disclosed in the present invention, comprise the application of transplantation, and stem cell therapy using non-human embryonic stem cells or non-human embryonic germ cells and neuronal adult stem cells, combined with any of the previously described cell- and gene therapeutic methods. Stem cells may be totipotent or pluripotent. They may also be organ-specific. Strategies for repairing diseased and/or damaged brain cells or tissue comprise (i) taking donor cells from an adult tissue. Nuclei of those cells are transplanted into unfertilized egg cells from which the genetic material has been removed. non-human Embryonic stem cells are isolated from the blastocyst stage of the cells which underwent somatic cell nuclear transfer. Use of differentiation factors then leads to a directed development of the stem cells to specialized cell types, preferably neuronal cells (Lanza et al., Nature Medicine 1999, 9: 975-977), or (ii) purifying adult stem cells, isolated from the central nervous system, or from bone marrow (mesenchymal stem cells), for in vitro expansion and subsequent grafting and transplantation, or (iii) directly inducing endogenous neural stem cells to proliferate, migrate, and differentiate into functional neurons (Peterson DA, Curr. Opin. Pharmacol. 2002, 2: 34-42). Adult neural stem cells are of great potential for repairing damaged or diseased brain tissues, as the germinal centers of the adult brain are basically free of neuronal damage or dysfunction (Colman A, Drug Discovery World 2001, 7: 66-71).

In preferred embodiments, the subject for treatment or prevention, according to the present invention, can be a human, an experimental animal, e.g. a mouse or a rat, a domestic animal, or a non-human primate. The experimental animal can be an animal model for a neurodegenerative disorder, e.g. a transgenic mouse and/or a knock-out mouse with an Alzheimer's-type neuropathology.

Further, the invention discloses a modulator of an activity, or a level, or both said activity and said level of at least one substance which is selected from the group consisting of (i) a gene coding for hTARPP, and/or (ii) a transcription product of the gene coding for hTARPP and/or (iii) a translation product of the gene coding for hTARPP, and/or (iv) a fragment, or derivative, or variant of (i) to (iii).

In addition, the invention discloses a pharmaceutical composition comprising said modulator and preferably a pharmaceutical carrier. Said carrier refers to a diluent, adjuvant, excipient, or vehicle with which the modulator is administered.

Disclosed is a modulator of an activity, or a level, or both said activity and said level of at least one substance which is selected from the group consisting of (i) a gene coding for hTARPP, and/or (ii) a transcription product of the gene coding for hTARPP, and/or (iii) a translation product of the gene coding for hTARPP, and/or (iv) a fragment, or derivative, or variant of (i) to (iii) for use in a pharmaceutical composition.

Further disclosed is the use of a modulator of an activity, or a level, or both said activity and said level of at least one substance which is selected from the group consisting of (i) a gene coding for hTARPP, and/or (ii) a transcription product of the gene coding for hTARPP and/or (iii) a translation product of the gene coding for hTARPP, and/or (iv) a fragment, or derivative, or variant of (i) to (iii) for a preparation of a medicament for treating or preventing a neurodegenerative disease, in particular Alzheimer's disease.

The present invention also discloses a kit comprising one or more containers filled with a therapeutically or prophylactically effective amount of said pharmaceutical composition.

Further, the invention discloses a recombinant, non-human animal comprising a non-native gene sequence coding for hTARPP, or a fragment, or a variant, or a derivative thereof. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing symptoms of neuropathology similar to a neurodegenerative disease, in particular Alzheimer's disease. Strategies and techniques for the generation and construction of such an animal are known to those of ordinary skill in the art (see e.g. Capecchi, Science 1989, 244: 1288-1292 and Hogan et al., 1994, Manipulating the Mouse Embryo: A Laboratory Manual, Cold Spring Harbor Laboratory Press, Cold Spring Harbor, New York and Jackson and Abbott, Mouse Genetics and Transgenics: A Practical Approach, Oxford University Press, Oxford, England, 1999). Also disclosed is the use of such a recombinant non-human animal as an animal model for investigating neurodegenerative diseases, in particular Alzheimer's disease. Such an animal may be useful for screening, testing and validating compounds, agents and modulators in the development of diagnostics and therapeutics to treat neurodegenerative diseases, in particular Alzheimer's disease.

The invention discloses an assay for screening for a modulator of neurodegenerative diseases, in particular Alzheimer's disease, or related diseases and disorders of one or more substances selected from the group consisting of (i) a gene coding for hTARPP, and/or (ii) a transcription product of the gene coding for hTARPP, and/or (iii) a translation product of the gene coding for hTARPP, and/or (iv) a fragment, or derivative, or variant of (i) to (iii). This screening method comprises (a) contacting a cell with a test compound, and (b) measuring the activity, or the level, or both the activity and the level of one or more substances recited in (i) to (iv), and (c) measuring the activity, or the level, or both the activity and the level of said substances in a control cell not contacted with said test compound, and (d) comparing the levels of the substance in the cells of step (b) and (c), wherein an alteration in the activity and/or level of said substances in the contacted cells indicates that the test compound is a modulator of said diseases and disorders.

Further, the invention discloses a screening assay for a modulator of neurodegenerative diseases, in particular Alzheimer's disease, or related diseases and disorders of one or more substances selected from the group consisting of (i) a gene coding for hTARPP, and/or (ii) a transcription product of the gene coding for hTARPP, and/or (iii) a translation product of the gene coding for hTARPP, and/or (iv) a fragment, or derivative, or variant of (i) to (iii), comprising (a) administering a test compound to a test animal which is predisposed to developing or has already developed symptoms of a neurodegenerative disease or related diseases or disorders, and (b) measuring the activity and/or level of one or more substances recited in (i) to (iv), and (c) measuring the activity and/or level of said substances in a matched control animal which is equally predisposed to developing or has already developed symptoms of said diseases and to which animal no such test compound has been administered, and (d) comparing the activity and/or level of the substance in the animals of step (b) and (c), wherein an alteration in the activity and/or level of substances in the test animal indicates that the test compound is a modulator of said diseases and disorders.

In a preferred embodiment, said test animal and/or said control animal is a recombinant non-human animal which expresses hTARPP, under the control of a transcriptional regulatory element which is not the native hTARPP gene transcriptional control regulatory element.

In another embodiment, the present invention provides a method for producing a medicament comprising the steps of (i) identifying a modulator of neurodegenerative diseases by a method of the aforementioned screening assays and (ii) admixing the modulator with a pharmaceutical carrier. However, said modulator may also be identifiable by other types of screening assays.

Additionally dislcosed is an assay for testing a compound, preferably for screening a plurality of compounds, for inhibition of binding between a ligand and hTARPP, or a fragment, or derivative, or variant thereof. Said screening assay comprises the steps of (i) adding a liquid suspension of said hTARPP, or a fragment, or variant, or derivative thereof, to a plurality of containers, and (ii) adding a compound or a plurality of compounds to be screened for said inhibition to said plurality of containers, and (iii) adding fluorescently labelled ligand to said containers, and (iv) incubating said hTARPP, or said fragment, or variant, or derivative thereof, and said compound or plurality of compounds, and said fluorescently labelled ligand, and (v) measuring the amounts of fluorescence associated with said hTARPP, or with said fragment, or variant, or derivative thereof, and (vi) determining the degree of inhibition by one or more of said compounds of binding of said ligand to said hTARPP, or said fragment, or variant, or derivative thereof. Instead of utilizing a fluorescently labelled ligand, it might in some aspects be preferred to use any other detectable label known to the person skilled in the art, e.g. radioactive labels, and detect it accordingly. Said method may be useful for the identification of novel compounds as well as for evaluating compounds which have been improved or otherwise optimized in their ability to inhibit the binding of a ligand to a gene product of the gene coding for hTARPP, or a fragment, or variant, or derivative thereof. One example of a fluorescent binding assay, in this case based on the use of carrier particles, is disclosed and described in patent application WO 00/52451. A further example is the competitive assay method as described in patent WO 02/01226. Preferred signal detection methods for screening assays of the instant invention are described in the following patent applications: WO 96/13744, WO 98/16814, WO 98/23942, WO 99/17086, WO 99/34195, WO 00/66985, WO 01/59436, WO 01/59416.

In one further embodiment, the present invention provides a method for producing a medicament comprising the steps of (i) identifying a compound as an inhibitor of binding between a ligand and a gene product of the gene coding for hTARPP by the aforementioned inhibitory binding assay and (ii) admixing the compound with a pharmaceutical carrier. However, said compound may also be identifiable by other types of screening assays.

The invention discloses an assay for testing a compound, preferably for screening a plurality of compounds to determine the degree of binding of said compounds to hTARPP, or to a fragment, a variant, or a derivative thereof. Said screening assay comprises (i) adding a liquid suspension of said hTARPP, or a fragment, or variant, or derivative thereof, to a plurality of containers, and (ii) adding a fluorescently labelled compound or a plurality of fluorescently labelled compounds to be screened for said binding to said plurality of containers, and (iii) incubating said hTARPP, or said fragment, or variant, or derivative thereof, and said fluorescently labelled compound or fluorescently labelled compounds, and (iv) measuring the amounts of fluorescence associated with said hTARPP, or with said fragment, or variant, or derivative thereof, and (v) determining the degree of binding by one or more of said compounds to said hTARPP, or said fragment, or variant, or derivative thereof. In this type of assay it might be preferred to use a fluorescent label. However, any other type of detectable label might also be employed. Said method may be useful for the identification of novel compounds as well as for evaluating compounds which have been improved or otherwise optimized in their ability to bind to hTARPP.

In one further embodiment, the present invention provides a method for producing a medicament comprising the steps of (i) identifying a compound as a binder to a gene product of the gene coding for hTARPP by the aforementioned binding assays and (ii) admixing the compound with a pharmaceutical carrier. However, said compound may also be identifiable by other types of screening assays.

In another embodiment, the present invention provides for a medicament obtainable by any of the methods according to the herein claimed screening assays. In one further embodiment, the instant invention provides for a medicament obtained by any of the methods according to the herein claimed screening assays.

Other features and advantages of the invention will be apparent from the following description of figures and examples which are illustrative only and not intended to limit the remainder of the disclosure in any way.

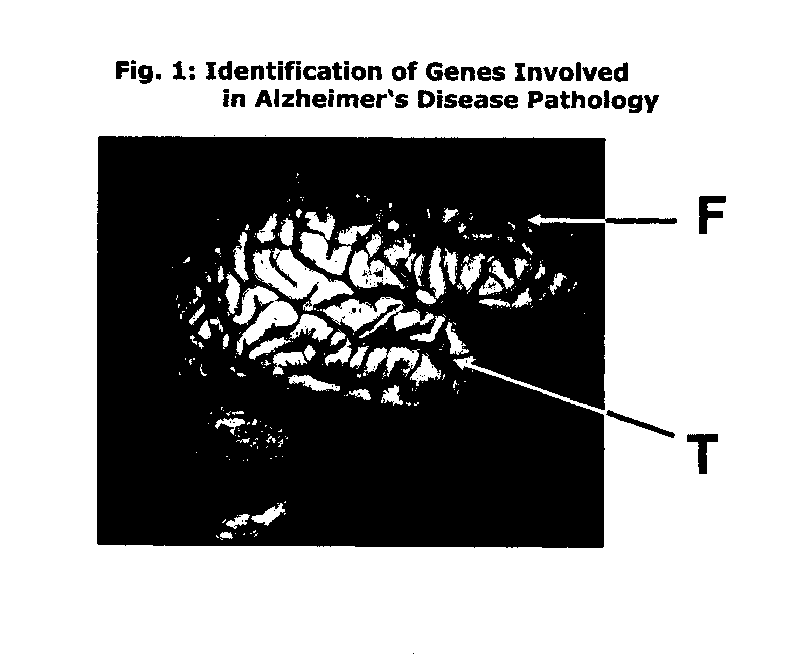

Figure 1 depicts the brain regions with selective vulnerability to neuronal loss and degeneration in Alzheimer's disease. Primarily, neurons within the inferior temporal lobe, the entorhinal cortex, the hippocampus, and the amygdala are subject to degenerative processes in Alzheimer's disease (Terry et al., Annals of Neurology 1981, 10:184-192). These brain regions are mostly involved in the processing of learning and memory functions. In contrast, neurons within the frontal cortex, the occipital cortex, and the cerebellum remain largely intact and preserved from neurodegenerative processes in Alzheimer's disease. Brain tissues from the frontal cortex (F), the temporal cortex (T), and the hippocampus (H) of Alzheimer's disease patients and healthy, age-matched control individuals were used for the herein disclosed examples. For illustrative purposes, the image of a normal healthy brain was taken from a publication by Strange *(*Brain Biochemistry and Brain Disorders, Oxford University Press, Oxford, 1992, p.4).

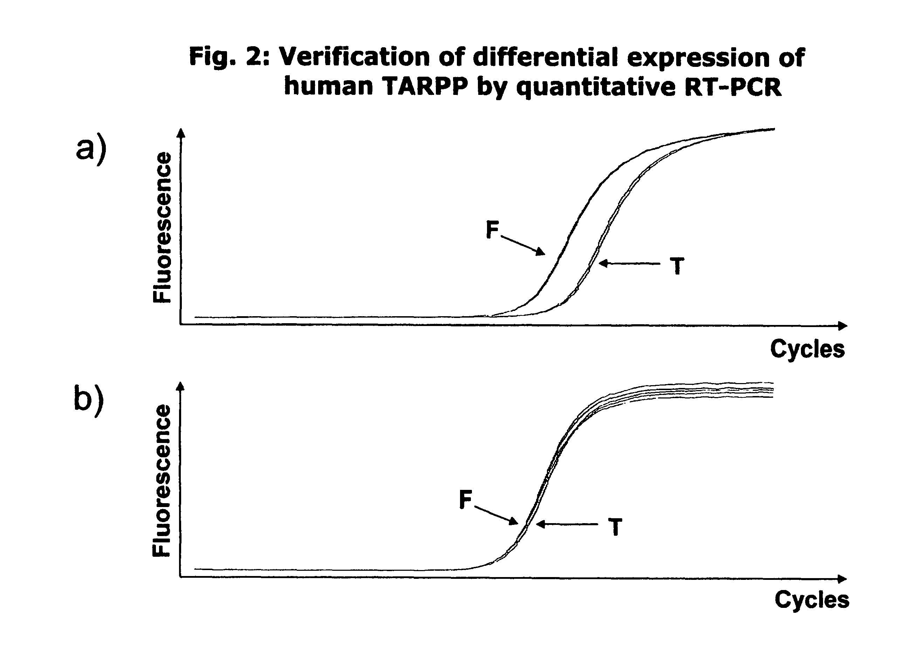

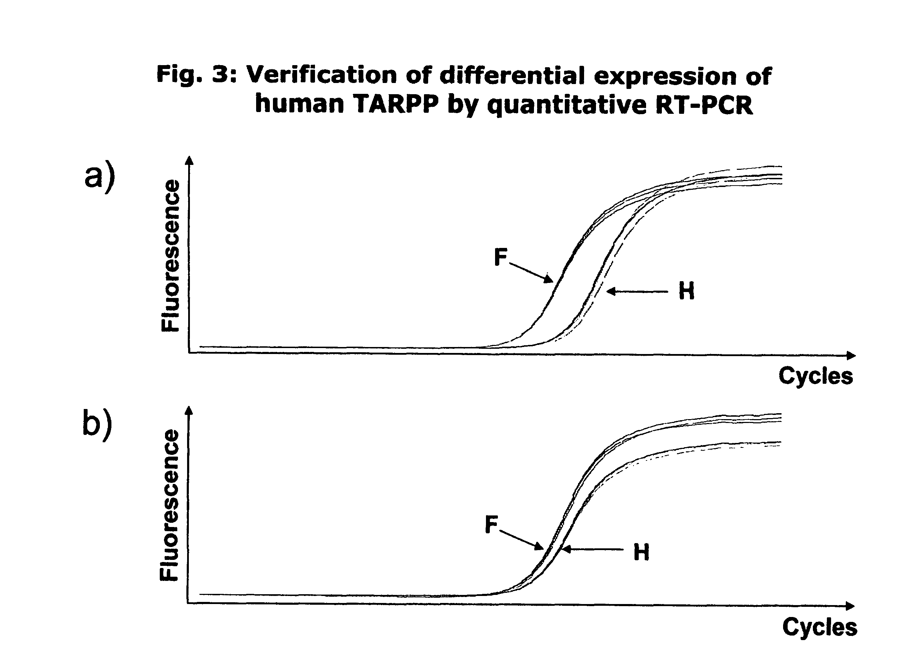

Figure 2 and 3 illustrate the verification of the differential expression of the human TARPP (hTARPP) gene in AD brain tissues by quantitative RT-PCR analysis. Quantification of RT-PCR products from RNA samples collected from the frontal cortex (F) and the temporal cortex (T) of AD patients (Figure 2a) and samples from the frontal cortex (F) and the hippocampus (H) of AD patients (Figure 3a) was performed by the LightCycler rapid thermal cycling technique. Likewise, samples of healthy, age-matched control individuals were compared (Figure 2b for frontal cortex and temporal cortex, Figure 3b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figure depicts the kinetics of amplification by plotting the cycle number against the amount of amplified material as measured by its fluorescence. Note that the amplification kinetics of hTARPP cDNAs from both, the frontal and temporal cortices of a normal control individual, and from the frontal cortex and hippocampus of a normal control individual, respectively, during the exponential phase of the reaction are juxtaposed (Figures 2b and 3b, arrowheads), whereas in Alzheimer's disease (Figures 2a and 3a, arrowheads) there is a significant separation of the corresponding curves, indicating a differential expression of the human TARPP gene in the respective analyzed brain regions.

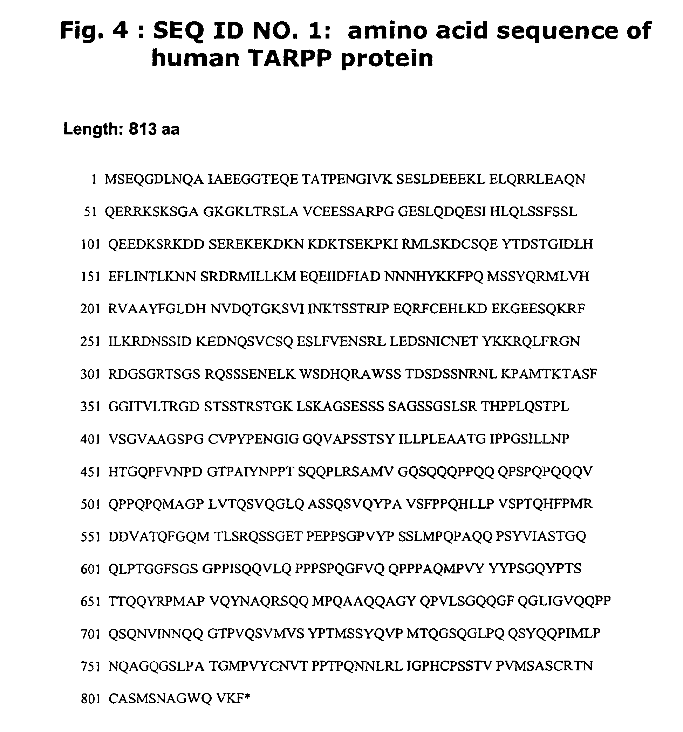

Figure 4 discloses the protein sequence of human TARPP (hTARPP); SEQ ID NO. 1. The full length human TARPP protein consists of 813 amino acids.

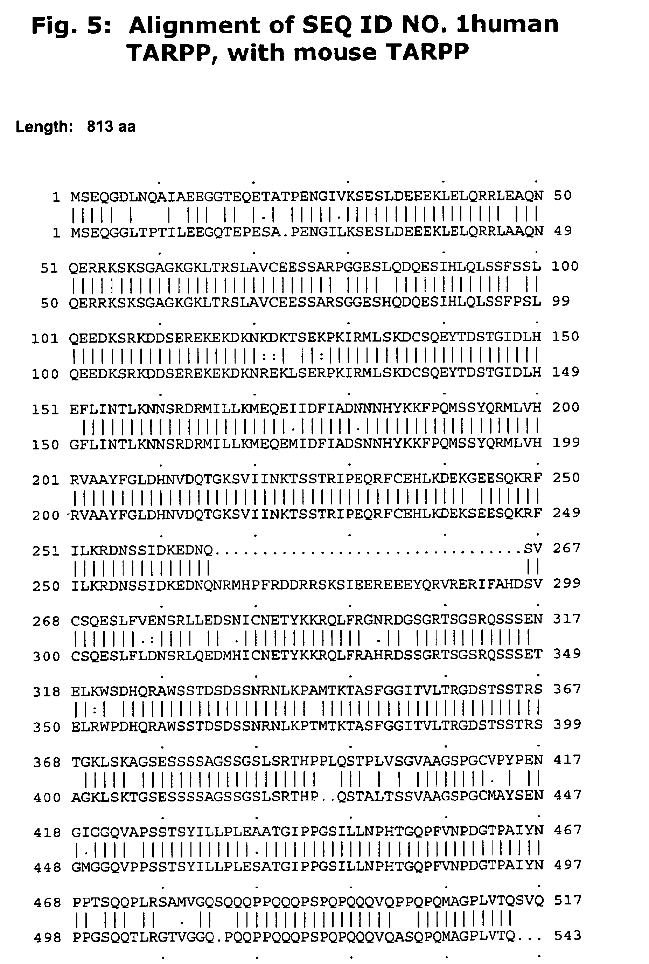

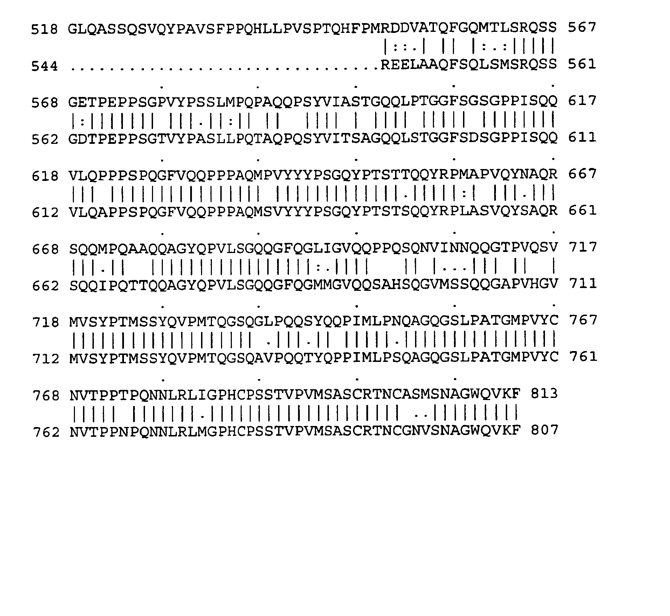

Figure 5 shows an alignment of the amino acid sequence of SEQ ID NO.1, hTARPP protein, with mouse (*Mus musculus*) TARPP amino acid sequence (GenBank accession number af324451).

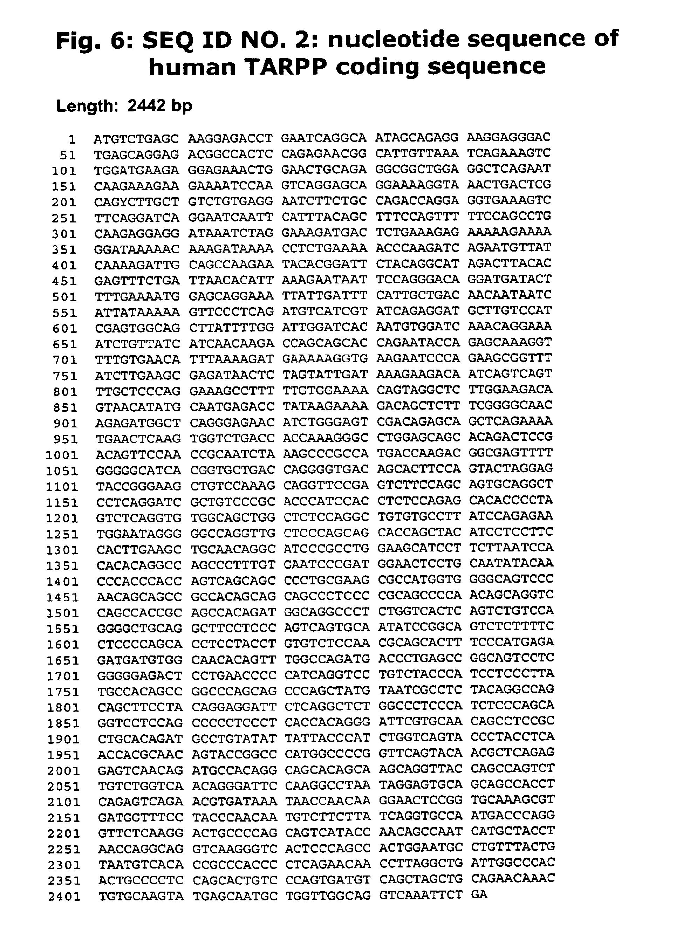

Figure 6 represents the nucleotide sequence of SEQ ID NO. 2, the coding sequence of the human TARPP gene, comprising 2442 nucleotides.

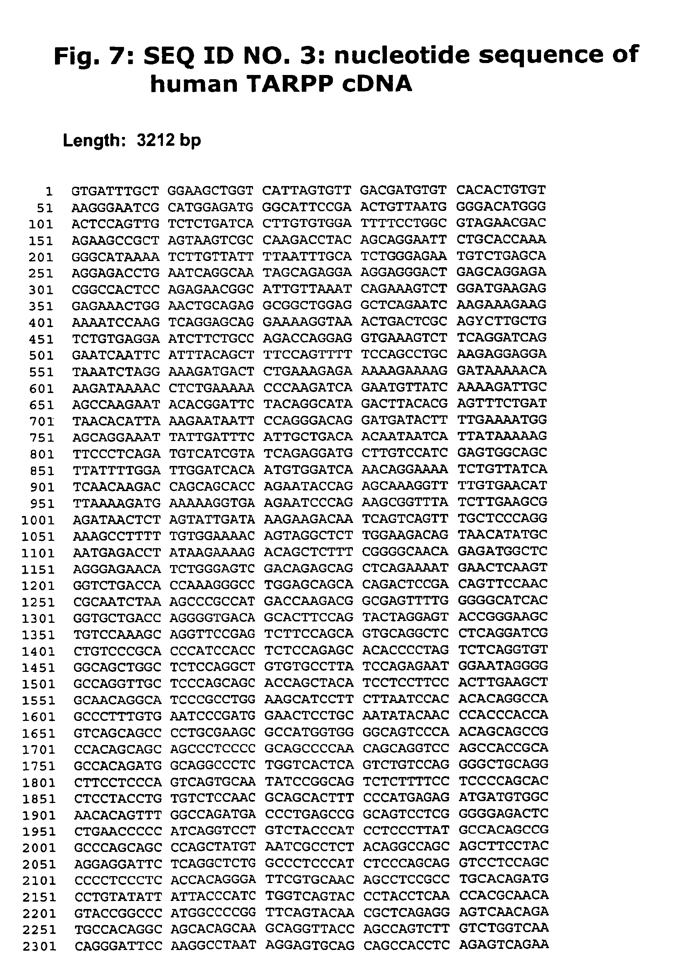

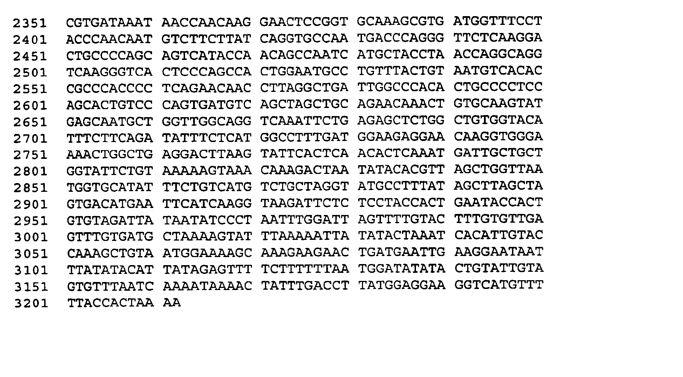

Figure 7 shows the nucleotide sequence of SEQ ID NO. 3, the hTARPP cDNA, comprising 3212 nucleotides. Primers used for quantitative PCR analysis are located from nucleotide 2471 to 2493 for the forward primer and from nucleotide 2518 to 2539 for the reverse primer.

Figure 8 depicts SEQ ID NO. 4, the nucleotide sequence of the 69 bp cDNA fragment, amplified with the primers used for quantitative PCR analysis. For the location of the primers refer to SEQ ID NO. 3, Figure 7.

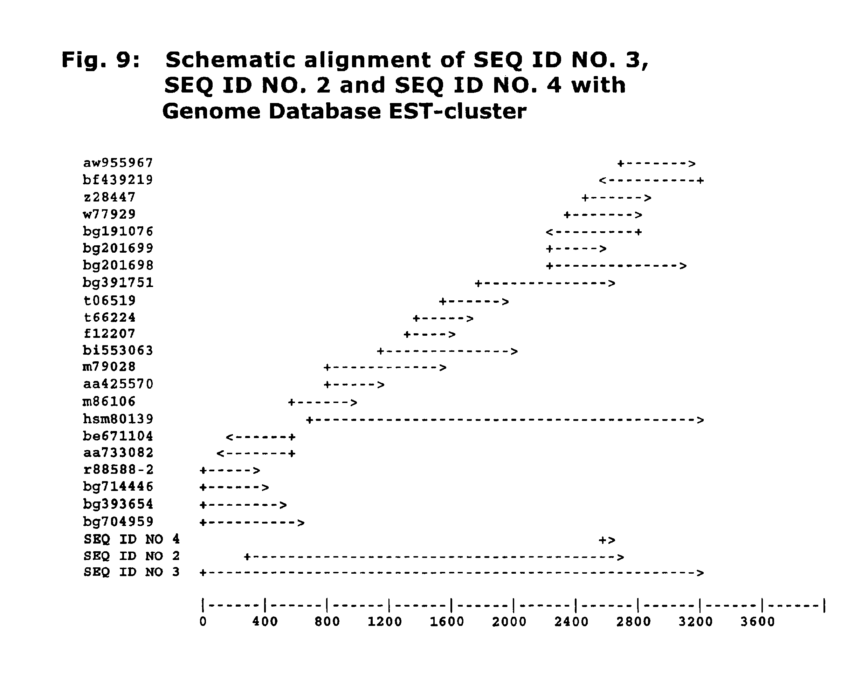

Figure 9 charts the schematic alignment of SEQ ID NO. 4, the hTARPP cDNA fragment, SEQ ID NO. 2, the coding sequence of the hTARPP gene, and SEQ ID NO. 3, the hTARPP gene nucleotide sequence derived from the alignment of human EST nucleotide sequences found in the GenBank genetic sequence database. EST numbers are written on the left side, all sequences are 5' to 3' directed.

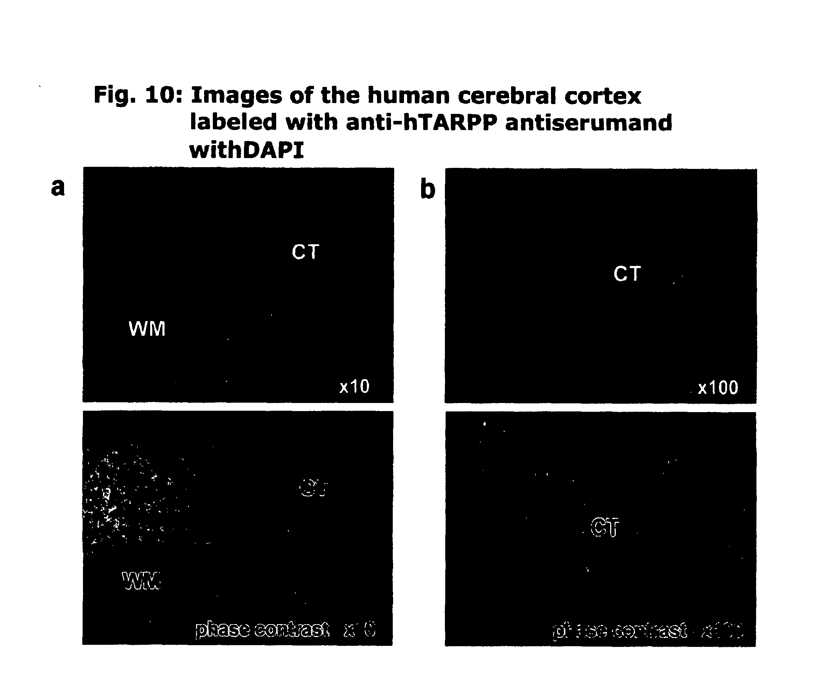

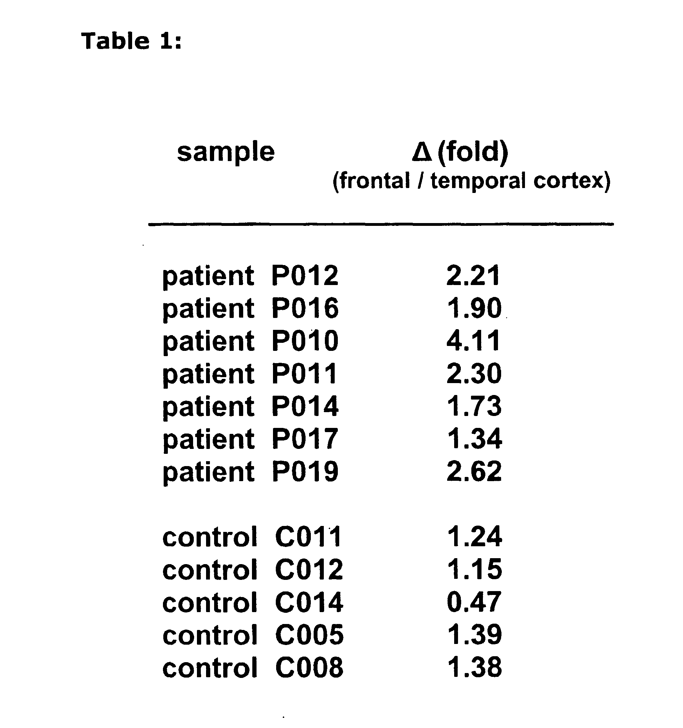

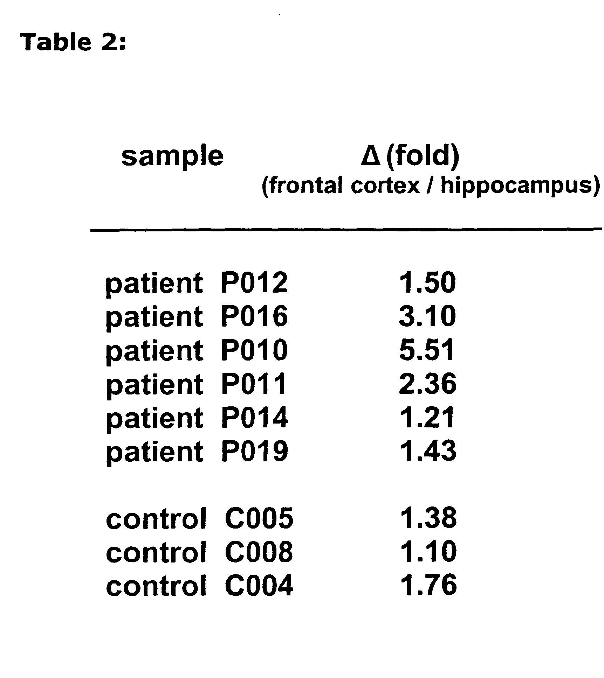

Figure 10 depicts human cerebral cortex labeled with an affinity-purified rabbit anti-hTARPP antiserum raised against a peptide corresponding to amino acids 566-580 (green signals). Strong immunoreactivity of human TARPP was detected in both, pre-central cortex (CT) and white matter (WM) (Figure 10a, low magnification). In the cortex, hTARPP is mainly detected in the cytoplasm of neuronal cell bodies and in some distal segments of neuronal processes (Figure 10b, high magnification). Moreover, axonal filaments and the cytoplasm of some glia cells were immuno-positive in the white matter. The same immunostaining pattern was observed by using another antiserum raised against a peptide mapping to amino acids 325-341 of hTARPP. Blue signals indicate nuclei stained with DAPI.

Table 1 lists expression levels in the frontal cortex relative to the temporal cortex for the transcription product of the hTARPP gene in seven Alzheimer's disease patients, herein identified by internal reference numbers P010, P011, P012, P014, P016, P017, P019 (1.34 to 4.11 fold) and five healthy, age-matched control individuals, herein identified by internal reference numbers C005, C008, C011, C012, C014 (0.47 to 1.39 fold). The values shown are reciprocal values according to the formula described herein (see below).

Table 2 lists the hTARPP gene expression levels in the frontal cortex relative to the hippocampus in six Alzheimer's disease patients, herein identified by internal reference numbers P010, P011, P012, P014, P016, P019 (1.21 to 5.51 fold) and three healthy, age-matched control individuals, herein identified by internal reference numbers C004, C005, C008 (1.10 to 1.76 fold). The values shown are reciprocal values according to the formula described herein (see below).

EXAMPLE I:

(i) <u>Brain tissue dissection from patients with Alzheimer's disease:</u>

Brain tissues from Alzheimer's disease patients and age-matched control subjects were collected within 6 hours post-mortem and immediately frozen on dry ice. Sample sections from each tissue were fixed in paraformaldehyde for histopathological confirmation of the diagnosis. Brain areas for differential expression analysis were identified (see Figure 1) and stored at - 80°C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality was determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were used to generate a melting curve with the LightCycler technology as described in the manufacturer's protocol (Roche).

(iii) Determination of differential expression by quantitative RT-PCR:

In order to identify changes in gene expression in different tissues we examined differential expression of the hTARPP gene using the LightCycler technology (Roche). This technique features rapid thermal cyling for the polymerase chain reaction as well as real-time measurement of fluorescent signals during amplification and therefore allows for highly accurate quantification of RT-PCR products by using a kinetic rather than an endpoint approach. The ratios of hTARPP cDNA from the temporal cortex and frontal cortex, and from the hippocampus and frontal cortex, respectively, were determined (relative quantification).

First, a standard curve was generated to determine the efficiency of the PCR with specific primers for the gene coding for hTARPP:

5'-ACAGCCAATCATGCTACCTAACC-3' and
5'-CAGTAAACAGGCATTCCAGTGG-3'.

PCR amplification (95 °C and 1 sec, 56 °C and 5 sec, and 72 °C and 5 sec) was performed in a volume of 20 μl containing Lightcycler-FastStart DNA Master SYBR Green I mix (containing FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and depending on the primers used, additional 3 mM $MgCl_2$. Melting curve analysis revealed a single peak at approximately 84.5°C with no visible primer dimers. Quality and size of the PCR product were determined with the DNA LabChip system (Agilent 2100 Bioanalyzer, Agilent Technologies). A single peak at the expected size of 69 bp for hTARPP was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTTTGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'- TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (12 bp). (4) GAPDH, using the specific primers 5'-CGTCATGGGTG-TGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGG-CTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for the gene coding for hTARPP and the five reference standard genes. The slopes and the intercepts of the standard curves (i.e. linear regressions) were calculated for all genes. In a second step, cDNAs from frontal cortex and temporal cortex, and from hippocampus and frontal cortex, respectively, were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \quad [\text{ng total brain cDNA}]$$

The values for temporal cortex and frontal cortex cDNAs of hTARPP, and the values for hippocampus and frontal cortex cDNAs of hTARPP, respectively, were normalized to cyclophilin B, and the ratio was calculated using the following formula:

$$\text{Ratio} = \frac{\text{hTARPP temporal [ng] / cyclophilin B temporal [ng]}}{\text{hTARPP frontal [ng] / cyclophilin B frontal [ng]}}$$

$$\text{Ratio} = \frac{\text{hTARPP hippocampus [ng] / cyclophilin B hippocampus [ng]}}{\text{hTARPP frontal [ng] / cyclophilin B frontal [ng]}}$$

In a third step, the set of reference standard genes was analyzed in parallel to determine the mean average value of the temporal to frontal ratios, and of the hippocampal to frontal ratios, respectively, of expression levels of the reference standard genes for each individual brain sample. As cyclophilin B was analyzed in step 2 and step 3, and the ratio from one gene to another gene remained constant in different runs, it was possible to normalize the values for hTARPP to the mean average value of the set of reference standard genes instead of normalizing to one single gene alone. The calculation was performed by dividing the ratios shown above by the deviation of cyclophilin B from the mean value of all housekeeping genes. The results of such quantitative RT-PCR analysis for hTARPP are shown in Figure 2 and 3.

(v) Sequence Analysis

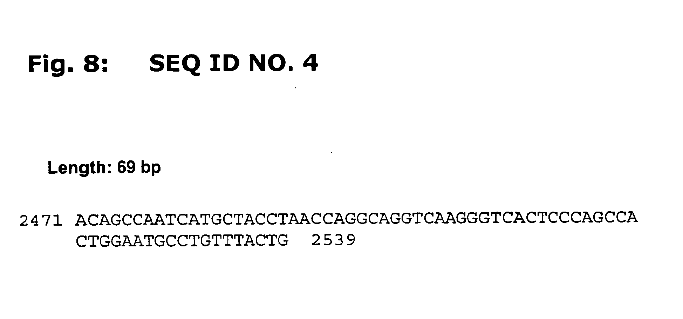

Searching the EST database of the GenBank database for sequence similarities to the identified differentially expressed human cDNA fragment (SEQ ID NO. 4), as stated in the present invention, it was found that SEQ ID NO. 4 is identical to portions of the human EST sequences hms80139 and bg201698 and others (shown in Figure 8). These human ESTs showed homology to mouse *(Mus musculus)* TARPP. Aligning human ESTs in addition to SEQ ID NO. 4, a complete EST cluster representing the hTARPP cDNA, SEQ ID NO. 3, was determined. The amino acid sequence of a large open reading frame, with the potential to encode a protein of 813 amino acid residues was deduced, SEQ ID NO. 1.

(vi) Immunohistochemistry:

For immunofluorescence staining of hTARPP in human brain, frozen sections were prepared from post-mortem pre-central gyrus of a donor person (Cryostat Leica CM3050S) and fixed in acetone at room temperature for 10 min. After washing in PBS, the sections were pre-incubated with blocking buffer (10% normal goat serum, 0.2% Triton X-100 in PBS) for 30min, and then incubated with affinity-purified rabbit anti-hTARPP antisera (1:20-1:40 diluted in blocking buffer, Eurogentec, Herstal, Belgium, custom-made) overnight at 4°C. After rinsing three times in 0.1 % Triton X-100/PBS, the sections were incubated with FITC-conjugated goat anti-rabbit IgG (1:150 diluted in 1% BSA/PBS) for 2 hours at room temperature, and then again washed in PBS. Staining of the nuclei was performed by incubation of the sections with 5μM DAPI in PBS for 3min (blue signal). In order to block the autofluoresence of lipofuscin in human brain, the sections were treated with 1% Sudan Black B in 70% ethanol for 2-10 min at room temperature and sequentially dipped in 70% ethanol, destilled water, and PBS. The sections were coverslipped by 'Vectrashield mounting medium' (Vector Laboratories, Burlingame, CA) and observed under an inverted microscope (IX81, Olympus Optical). The digital images were captured with the appropriate software (AnalySiS, Olympus Optical).

## Claims

**1.** An isolated nucleic acid encoding a protein molecule shown in SEQ ID NO. 1.

**2.** An isolated nucleic acid molecule of claim 1, wherein the nucleic acid molecule is a DNA molecule.

3. An isolated nucleic acid molecule of claim 2, wherein the nucleic acid molecule is a cDNA molecule, in particular a cDNA molecule comprising a nucleotide sequence shown in SEQ ID NO. 2 or SEQ ID NO.3.

4. An isolated DNA molecule capable of hybridizing with the complement of the cDNA described in SEQ ID NO. 2 or SEQ ID NO. 3 under stringent condition.

5. A vector comprising a nucleic acid molecule according to one of claims 1 to 4.

6. A vector according to claim 5 wherein said vector is a plasmid, a virus or a bacteriophage.

7. A cell transformed with a nucleic acid molecule according to one of claims 1 to 4, wherein said cell is in particular a bacterial cell, a yeast cell a mammalian cell, or an insect cell, provided that the cell is not derived from human embryo.

8. A protein molecule shown in SEQ ID NO. 1.

9. A protein molecule shown in SEQ ID NO. 1 for use as a diagnostic target for detecting Alzheimer's disease.

10. Use of an antibody specifically immunoreactive with an immunogen, wherein said immunogen is a protein molecule shown in SEQ ID NO. 1, , for detecting the Alzheimer's disease state of a cell in a sample from a subject, comprising immunocytochemical staining of said cell with said antibody, wherein an altered degree of staining, or an altered staining pattern in said cell compared to a cell representing a known health status indicates the Alzheimer's disease state of said cell.

11. A method of diagnosing or prognosticating Alzheimer's disease in a subject, or determining whether a subject is at increased risk of developing said disease, comprising:

determining a level and/or an activity of

(i) a transcription product of the gene coding for hTARPP, and/or
(ii) a translation product of the gene coding for hTARPP,

in a brain sample obtained from said subject and comparing said level and/or said activity to a reference value representing a known disease or health status, thereby diagnosing or prognosticating said disease in said subject, or determining whether said subject is at increased risk of developing said disease.

12. The method according to claim 11 wherein said reference value is that of a level and/or an activity of

(i) a transcription product of the gene coding for hTARPP, and/or
(ii) a translation product of the gene coding for hTARPP, in a sample from a subject not suffering from Alzheimer's disease.

13. The method according to any of claims 11 or 12 wherein an alteration in the level and/or activity of a transcription product of the gene coding for hTARPP and/or a translation product of the gene coding for hTARPP in a sample cell, or tissue, or body fluid, in particular cerebrospinal fluid, from said subject relative to a reference value representing a known health status indicates a diagnosis, or prognosis, or increased risk of Alzheimer's disease in said subject.

**Patentansprüche**

1. Isolierte Nucleinsäure, die ein in SEQ ID Nr. 1 gezeigtes Proteinmolekül codiert.

2. Isoliertes Nucleinsäuremolekül gemäß Anspruch 1, wobei das Nucleinsäuremolekül ein DNA-Molekül ist.

3. Isoliertes Nucleinsäuremolekül gemäß Anspruch 2, wobei das Nucleinsäuremolekül ein cDNA-Molekül ist, insbesondere ein cDNA-Molekül, das eine in SEQ ID Nr. 2 oder SEQ ID Nr. 3 gezeigte Nucleotidsequenz umfasst.

4. Isoliertes DNA-Molekül, das unter stringenten Bedingungen mit dem Komplement der in SEQ ID Nr. 2 oder SEQ ID Nr. 3 beschriebenen cDNA hybridisieren kann.

5. Vektor, der ein Nucleinsäuremolekül gemäß einem der Ansprüche 1 bis 4 umfasst.

6. Vektor gemäß Anspruch 5, wobei der Vektor ein Plasmid, ein Virus oder ein Bakteriophage ist.

7. Zelle, die mit einem Nucleinsäuremolekül gemäß einem der Ansprüche 1 bis 4 transformiert ist, wobei die Zelle insbesondere eine Bakterienzelle, eine Hefezelle, eine Säugerzelle oder eine Insektenzelle ist, mit der Maßgabe, dass die Zelle nicht von einem humanen Embryo stammt.

8. Proteinmolekül, das in SEQ ID Nr. 1 gezeigt ist.

9. Proteinmolekül, das in SEQ ID Nr. 1 gezeigt ist, zur Verwendung als diagnostisches Target zum Nachweis von Alzheimer-Krankheit.

10. Verwendung eines Antikörpers, der gegenüber einem Immunogen spezifisch immunreaktiv ist, wobei das Immunogen ein in SEQ ID Nr. 1 gezeigtes Proteinmolekül ist, zum Nachweis des Alzheimer-Krankheit-Status einer Zelle in einer Probe von einem Patienten, umfassend das immuncytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitsstatus repräsentiert, den Alzheimer-Krankheit-Status der Zelle anzeigt.

11. Verfahren zum Diagnostizieren oder Prognostizieren der Alzheimer-Krankheit bei einem Patienten oder zum Bestimmen, ob ein Patient ein erhöhtes Risiko hat, diese Krankheit zu entwickeln, umfassend:

    Bestimmen einer Konzentration und/oder einer Aktivität von:

    (i) einem Transcriptionsprodukt des Gens, das für hTARPP codiert; und/oder
    (ii) einem Translationsprodukt des Gens, das für hTARPP codiert;

    in einer von dem Patienten erhaltenen Gehirnprobe und Vergleichen der Konzentration und/oder der Aktivität mit einem Referenzwert, der einen bekannten Krankheits- oder Gesundheitsstatus darstellt, und **dadurch** Diagnostizieren oder Prognostizieren der Krankheit bei dem Patienten oder Bestimmen, ob der Patient ein erhöhtes Risiko hat, die Krankheit zu entwickeln.

12. Verfahren gemäß Anspruch 11, wobei der Referenzwert der Referenzwert einer Konzentration und/oder Aktivität

    (i) eines Transcriptionsprodukts des Gens, das für hTARPP codiert, und/oder
    (ii) eines Translationsprodukts des Gens, das für hTARPP codiert,
    in einer Probe von einem Patienten, der nicht an Alzheimer-Krankheit leidet, ist.

13. Verfahren gemäß einem der Ansprüche 11 oder 12, wobei eine Veränderung der Konzentration und/oder Aktivität eines Transcriptionsprodukts des Gens, das für hTARPP codiert, und/oder eines Translationsprodukts des Gens, das für hTARPP codiert, in einer Probenzelle oder einem Gewebe oder einer Körperflüssigkeit, insbesondere Liquor, von dem Patienten relativ zu einem Referenzwert, der einen bekannten Gesundheitsstatus darstellt, eine Diagnose oder Prognose oder ein erhöhtes Risiko der Alzheimer-Krankheit bei dem Patienten anzeigt.

## Revendications

1. Acide nucléique isolé codant pour une molécule de protéine présentée dans SEQ ID NO: 1.

2. Molécule d'acide nucléique isolée selon la revendication 1, dans laquelle la molécule d'acide nucléique est une molécule d'ADN.

3. Molécule d'acide nucléique isolée selon la revendication 2, dans laquelle la molécule d'acide nucléique est une molécule d'ADNc, en particulier une molécule d'ADNc comprenant une séquence de nucléotides présentée dans SEQ ID NO : 2 et SEQ ID NO : 3.

4. Molécule d'ADN isolée capable de s'hybrider avec le complément de l'ADNc décrit dans SEQ ID NO : 2 ou SEQ ID NO : 3 dans des conditions stringentes.

**5.** Vecteur comprenant une molécule d'acide nucléique selon l'une quelconque des revendications 1 à 4.

**6.** Vecteur selon la revendication 5, dans lequel ledit vecteur est un plasmide, un virus ou un bactériophage.

**7.** Cellule transformée avec une molécule d'acide nucléique selon l'une quelconque des revendications 1 à 4, dans laquelle ladite cellule est en particulier une cellule bactérienne, une cellule de levure, une cellule de mammifère ou une cellule d'insecte, à condition que la cellule ne soit pas dérivée d'un embryon humain.

**8.** Molécule de protéine présentée dans SEQ ID NO : 1.

**9.** Molécule de protéine présentée dans SEQ ID NO : 1, pour utilisation comme cible de diagnostic pour détecter la maladie d'Alzheimer.

**10.** Utilisation d'un anticorps spécifiquement immunoréactif avec un immunogène, dans laquelle ledit immunogène est une molécule de protéine présentée dans SEQ ID NO : 1, pour détecter l'état de la maladie d'Alzheimer d'une cellule dans un échantillon provenant d'un sujet, comprenant la coloration immunocytochimique de ladite cellule avec ledit anticorps, où un degré de coloration modifié, ou un motif de coloration modifié dans ladite cellule par rapport à une cellule représentant un état de santé connu, indique l'état de la maladie d'Alzheimer de ladite cellule.

**11.** Procédé de diagnostic ou de pronostic de la maladie d'Alzheimer chez un sujet, ou consistant à déterminer si un sujet présente un risque accru de développer ladite maladie, comprenant l'étape consistant à :

déterminer un taux et/ou une activité

(i) d'un produit de transcription du gène codant pour hTARPP, et/ou
(ii) d'un produit de traduction du gène codant pour hTARPP,

dans un échantillon de cerveau obtenu à partir dudit sujet et comparer ledit taux et/ou ladite activité à une valeur de référence représentant une maladie connue ou un état de santé connu, permettant ainsi de diagnostiquer ou pronostiquer ladite maladie chez ledit sujet, ou de déterminer si ledit sujet présente un risque accru de développer ladite maladie.

**12.** Procédé selon la revendication 11, dans lequel ladite valeur de référence est celle d'un taux et/ou d'une activité

(i) d'un produit de transcription du gène codant pour hTARPP, et/ou
(ii) d'un produit de traduction du gène codant pour hTARPP,
dans un échantillon provenant d'un sujet ne souffrant pas de la maladie d'Alzheimer.

**13.** Procédé selon l'une quelconque des revendications 11 ou 12, dans lequel une modification du taux et/ou de l'activité d'un produit de transcription du gène codant pour hTARPP et/ou d'un produit de traduction du gène codant pour hTARPP dans une cellule échantillon, ou un tissu ou un fluide corporel, en particulier le liquide céphalo-rachidien, provenant dudit sujet, relativement à une valeur de référence représentant un état de santé connu, indique un diagnostic, ou un pronostic, ou un risque accru de maladie d'Alzheimer pour ledit sujet.

**Fig. 1: Identification of Genes Involved in Alzheimer's Disease Pathology**

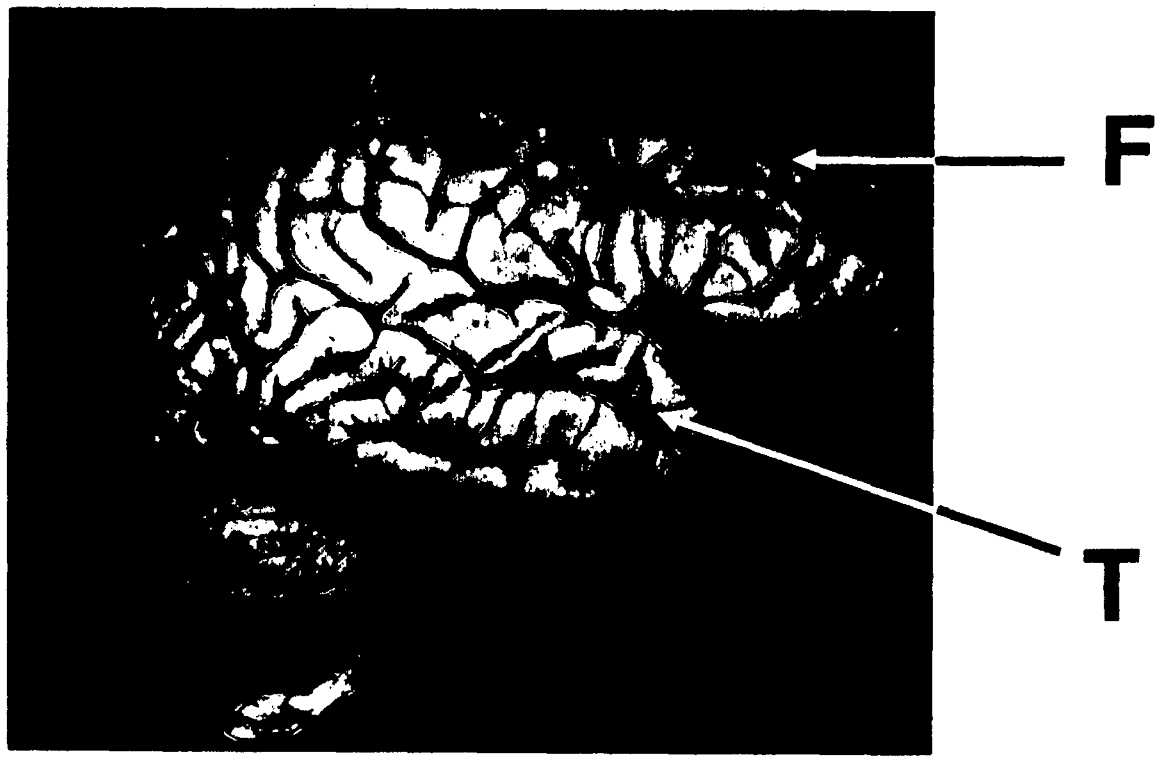

**Fig. 2: Verification of differential expression of human TARPP by quantitative RT-PCR**

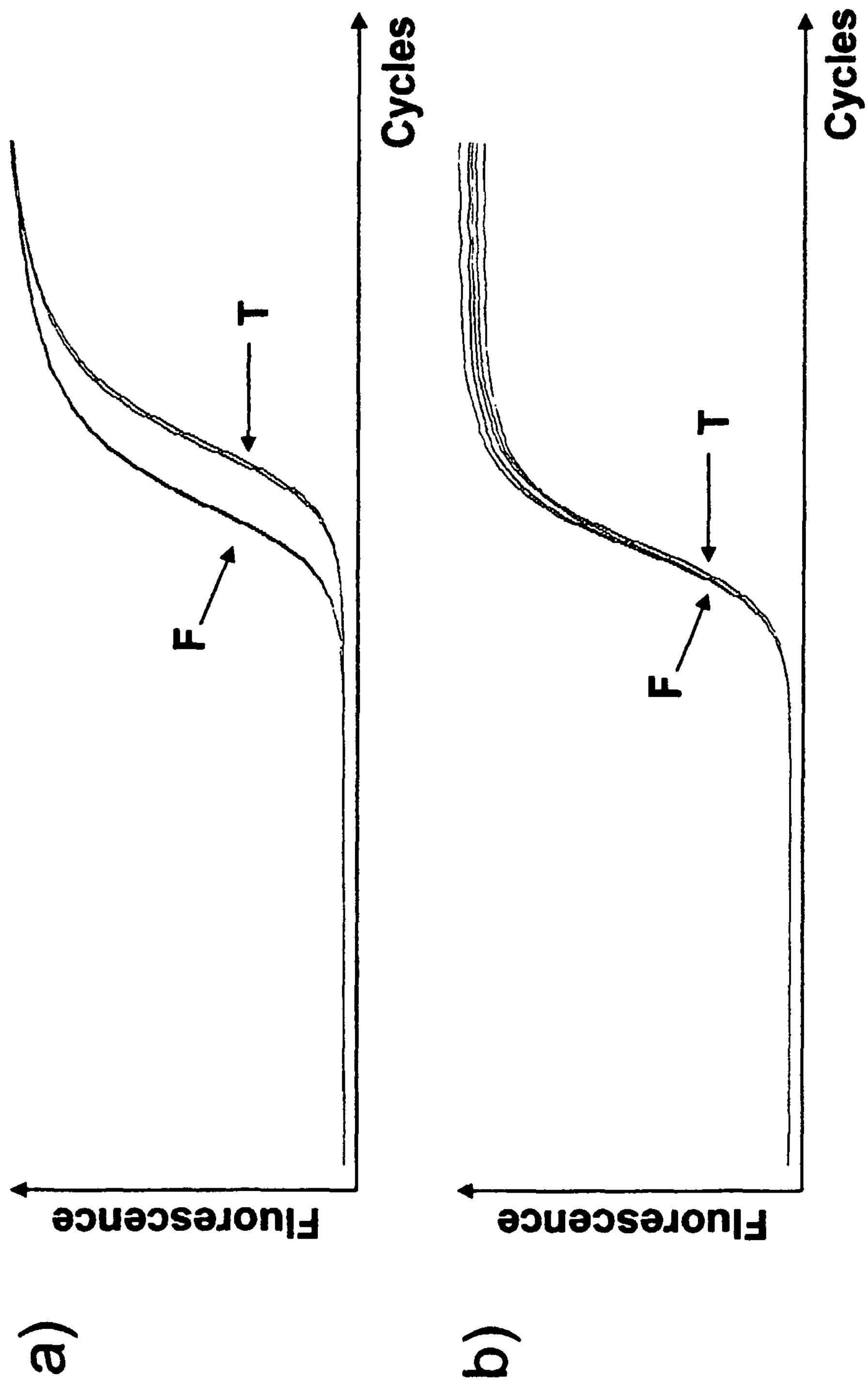

**Fig. 3: Verification of differential expression of human TARPP by quantitative RT-PCR**

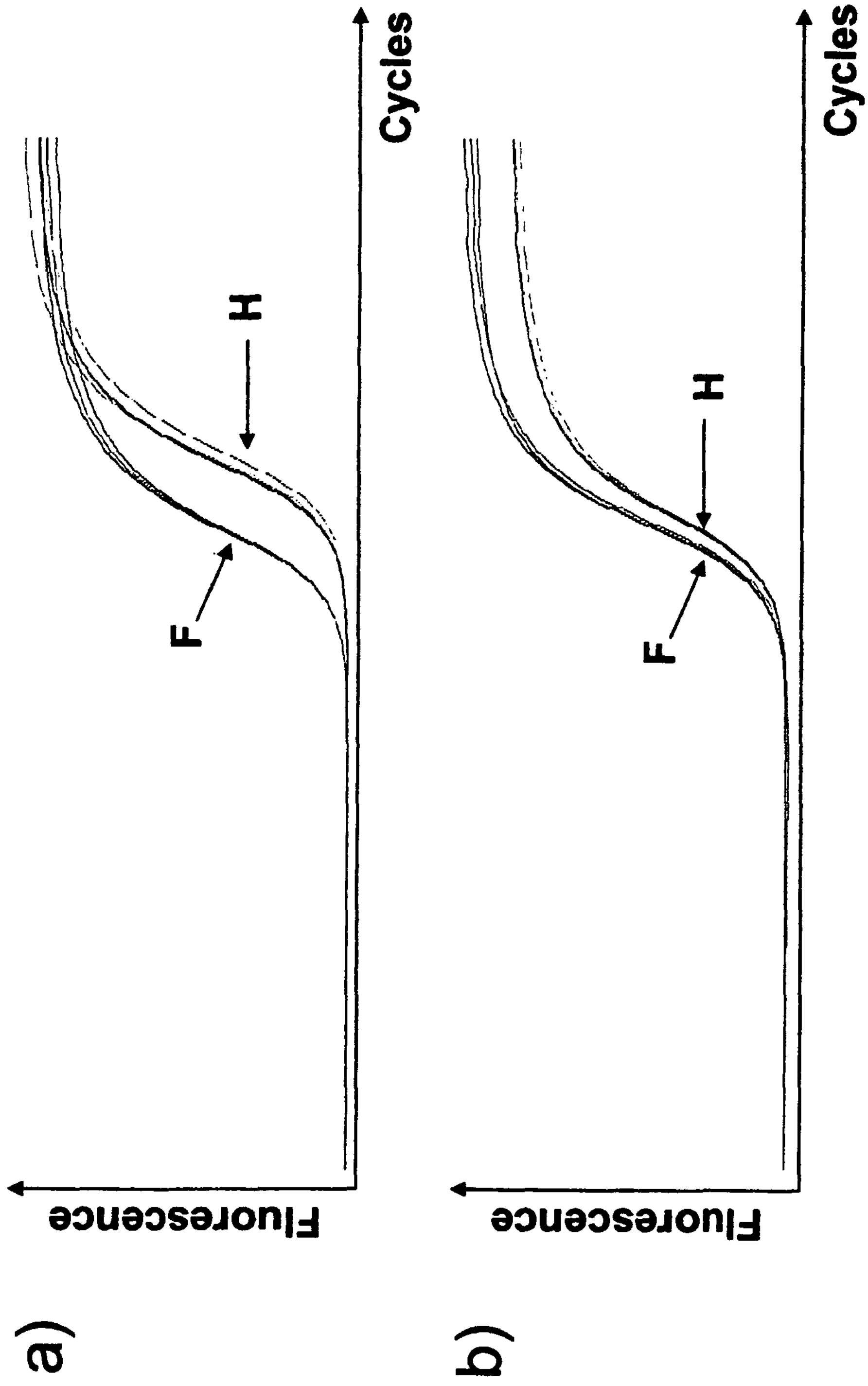

# Fig. 4 : SEQ ID NO. 1: amino acid sequence of human TARPP protein

**Length: 813 aa**

1 MSEQGDLNQA IAEEGGTEQE TATPENGIVK SESLDEEEKL ELQRRLEAQN

51 QERRKSKSGA GKGKLTRSLA VCEESSARPG GESLQDQESI HLQLSSFSSL

101 QEEDKSRKDD SEREKEKDKN KDKTSEKPKI RMLSKDCSQE YTDSTGIDLH

151 EFLINTLKNN SRDRMILLKM EQEIIDFIAD NNNHYKKFPQ MSSYQRMLVH

201 RVAAYFGLDH NVDQTGKSVI INKTSSTRIP EQRFCEHLKD EKGEESQKRF

251 ILKRDNSSID KEDNQSVCSQ ESLFVENSRL LEDSNICNET YKKRQLFRGN

301 RDGSGRTSGS RQSSSENELK WSDHQRAWSS TDSDSSNRNL KPAMTKTASF

351 GGITVLTRGD STSSTRSTGK LSKAGSESSS SAGSSGSLSR THPPLQSTPL

401 VSGVAAGSPG CVPYPENGIG GQVAPSSTSY ILLPLEAATG IPPGSILLNP

451 HTGQPFVNPD GTPAIYNPPT SQQPLRSAMV GQSQQQPPQQ QPSPQPQQQV

501 QPPQPQMAGP LVTQSVQGLQ ASSQSVQYPA VSFPPQHLLP VSPTQHFPMR

551 DDVATQFGQM TLSRQSSGET PEPPSGPVYP SSLMPQPAQQ PSYVIASTGQ

601 QLPTGGFSGS GPPISQQVLQ PPPSPQGFVQ QPPPAQMPVY YYPSGQYPTS

651 TTQQYRPMAP VQYNAQRSQQ MPQAAQQAGY QPVLSGQQGF QGLIGVQQPP

701 QSQNVINNQQ GTPVQSVMVS YPTMSSYQVP MTQGSQGLPQ QSYQQPIMLP

751 NQAGQGSLPA TGMPVYCNVT PPTPQNNLRL IGPHCPSSTV PVMSASCRTN

801 CASMSNAGWQ VKF*

# Fig. 5: Alignment of SEQ ID NO. 1human TARPP, with mouse TARPP

**Length: 813 aa**

```
                  .         .         .         .         .
   1 MSEQGDLNQAIAEEGGTEQETATPENGIVKSESLDEEEKLELQRRLEAQN  50
     ||||| |   | ||| || |.| |||||.||||||||||||||||| |||
   1 MSEQGGLTPTILEEGQTEPESA.PENGILKSESLDEEEKLELQRRLAAQN  49
                  .         .         .         .         .
  51 QERRKSKSGAGKGKLTRSLAVCEESSARPGGESLQDQESIHLQLSSFSSL 100
     |||||||||||||||||||||||||||| |||| ||||||||||||| ||
  50 QERRKSKSGAGKGKLTRSLAVCEESSARSGGESHQDQESIHLQLSSFPSL  99
                  .         .         .         .         .
 101 QEEDKSRKDDSEREKEKDKNKDKTSEKPKIRMLSKDCSQEYTDSTGIDLH 150
     ||||||||||||||||||||::| ||:|||||||||||||||||||||||
 100 QEEDKSRKDDSEREKEKDKNREKLSERPKIRMLSKDCSQEYTDSTGIDLH 149
                  .         .         .         .         .
 151 EFLINTLKNNSRDRMILLKMEQEIIDFIADNNNHYKKFPQMSSYQRMLVH 200
      ||||||||||||||||||||||.||||||.|||||||||||||||||||
 150 GFLINTLKNNSRDRMILLKMEQEMIDFIADSNNHYKKFPQMSSYQRMLVH 199
                  .         .         .         .         .
 201 RVAAYFGLDHNVDQTGKSVIINKTSSTRIPEQRFCEHLKDEKGEESQKRF 250
     |||||||||||||||||||||||||||||||||||||||||| |||||||
 200 RVAAYFGLDHNVDQTGKSVIINKTSSTRIPEQRFCEHLKDEKSEESQKRF 249
                  .         .         .         .         .
 251 ILKRDNSSIDKEDNQ.................................SV 267
     |||||||||||||||                                 ||
 250 ILKRDNSSIDKEDNQNRMHPFRDDRRSKSIEEREEEYQRVRERIFAHDSV 299
                  .         .         .         .         .
 268 CSQESLFVENSRLLEDSNICNETYKKRQLFRGNRDGSGRTSGSRQSSSEN 317
     |||||||.:|||| || .||||||||||||| .|| ||||||||||||| 
 300 CSQESLFLDNSRLQEDMHICNETYKKRQLFRAHRDSSGRTSGSRQSSSET 349
                  .         .         .         .         .
 318 ELKWSDHQRAWSSTDSDSSNRNLKPAMTKTASFGGITVLTRGDSTSSTRS 367
     ||:| |||||||||||||||||||| ||||||||||||||||||||||||
 350 ELRWPDHQRAWSSTDSDSSNRNLKPTMTKTASFGGITVLTRGDSTSSTRS 399
                  .         .         .         .         .
 368 TGKLSKAGSESSSSAGSSGSLSRTHPPLQSTPLVSGVAAGSPGCVPYPEN 417
      ||||| |||||||||||||||||||  ||| | | ||||||||. | ||
 400 AGKLSKTGSESSSSAGSSGSLSRTHP..QSTALTSSVAAGSPGCMAYSEN 447
                  .         .         .         .         .
 418 GIGGQVAPSSTSYILLPLEAATGIPPGSILLNPHTGQPFVNPDGTPAIYN 467
     |.|||| ||||||||||||.||||||||||||||||||||||||||||||
 448 GMGGQVPPSSTSYILLPLESATGIPPGSILLNPHTGQPFVNPDGTPAIYN 497
                  .         .         .         .         .
 468 PPTSQQPLRSAMVGQSQQQPPQQQPSPQPQQQVQPPQPQMAGPLVTQSVQ 517
     || ||| ||  . ||  |||||||||||||||||  |||||||||||   
 498 PPGSQQTLRGTVGGQ.PQQPPQQQPSPQPQQQVQASQPQMAGPLVTQ... 543
                  .         .         .         .         .
```

```
518 GLQASSQSVQYPAVSFPPQHLLPVSPTQHFPMRDDVATQFGQMTLSRQSS 567
                                    |::.| || |:.:|||||
544 ................................REELAAQFSQLSMSRQSS 561
             .         .         .         .         .
568 GETPEPPSGPVYPSSLMPQPAQQPSYVIASTGQQLPTGGFSGSGPPISQQ 617
    |:||||||| |||.||:|| ||  |||| | |||| ||||| ||||||||
562 GDTPEPPSGTVYPASLLPQTAQPQSYVITSAGQQLSTGGFSDSGPPISQQ 611
             .         .         .         .         .
618 VLQPPPSPQGFVQQPPPAQMPVYYYPSGQYPTSTTQQYRPMAPVQYNAQR 667
    ||| |||||||||||||||| |||||||||||||.|||||:| |||.|||
612 VLQAPPSPQGFVQQPPPAQMSVYYYPSGQYPTSTSQQYRPLASVQYSAQR 661
             .         .         .         .         .
668 SQQMPQAAQQAGYQPVLSGQQGFQGLIGVQQPPQSQNVINNQQGTPVQSV 717
    |||.||  |||||||||||||||||:.||||   || |...||| ||  |
662 SQQIPQTTQQAGYQPVLSGQQGFQGMMGVQQSAHSQGVMSSQQGAPVHGV 711
             .         .         .         .         .
718 MVSYPTMSSYQVPMTQGSQGLPQQSYQQPIMLPNQAGQGSLPATGMPVYC 767
    ||||||||||||||||||| .|||.|| |||||.||||||||||||||||
712 MVSYPTMSSYQVPMTQGSQAVPQQTYQPPIMLPSQAGQGSLPATGMPVYC 761
             .         .         .         .
768 NVTPPTPQNNLRLIGPHCPSSTVPVMSASCRTNCASMSNAGWQVKF 813
    ||||| |||||||.|||||||||||||||||||| ..|||||||||
762 NVTPPNPQNNLRLMGPHCPSSTVPVMSASCRTNCGNVSNAGWQVKF 807
```

# Fig. 6: SEQ ID NO. 2: nucleotide sequence of human TARPP coding sequence

**Length: 2442 bp**

```
   1 ATGTCTGAGC AAGGAGACCT GAATCAGGCA ATAGCAGAGG AAGGAGGGAC
  51 TGAGCAGGAG ACGGCCACTC CAGAGAACGG CATTGTTAAA TCAGAAAGTC
 101 TGGATGAAGA GGAGAAACTG GAACTGCAGA GGCGGCTGGA GGCTCAGAAT
 151 CAAGAAAGAA GAAAATCCAA GTCAGGAGCA GGAAAAGGTA AACTGACTCG
 201 CAGYCTTGCT GTCTGTGAGG AATCTTCTGC CAGACCAGGA GGTGAAAGTC
 251 TTCAGGATCA GGAATCAATT CATTTACAGC TTTCCAGTTT TTCCAGCCTG
 301 CAAGAGGAGG ATAAATCTAG GAAAGATGAC TCTGAAAGAG AAAAAGAAAA
 351 GGATAAAAAC AAAGATAAAA CCTCTGAAAA ACCCAAGATC AGAATGTTAT
 401 CAAAAGATTG CAGCCAAGAA TACACGGATT CTACAGGCAT AGACTTACAC
 451 GAGTTTCTGA TTAACACATT AAAGAATAAT TCCAGGGACA GGATGATACT
 501 TTTGAAAATG GAGCAGGAAA TTATTGATTT CATTGCTGAC AACAATAATC
 551 ATTATAAAAA GTTCCCTCAG ATGTCATCGT ATCAGAGGAT GCTTGTCCAT
 601 CGAGTGGCAG CTTATTTTGG ATTGGATCAC AATGTGGATC AAACAGGAAA
 651 ATCTGTTATC ATCAACAAGA CCAGCAGCAC CAGAATACCA GAGCAAAGGT
 701 TTTGTGAACA TTTAAAAGAT GAAAAAGGTG AAGAATCCCA GAAGCGGTTT
 751 ATCTTGAAGC GAGATAACTC TAGTATTGAT AAAGAAGACA ATCAGTCAGT
 801 TTGCTCCCAG GAAAGCCTTT TTGTGGAAAA CAGTAGGCTC TTGGAAGACA
 851 GTAACATATG CAATGAGACC TATAAGAAAA GACAGCTCTT TCGGGGCAAC
 901 AGAGATGGCT CAGGGAGAAC ATCTGGGAGT CGACAGAGCA GCTCAGAAAA
 951 TGAACTCAAG TGGTCTGACC ACCAAAGGGC CTGGAGCAGC ACAGACTCCG
1001 ACAGTTCCAA CCGCAATCTA AAGCCCGCCA TGACCAAGAC GGCGAGTTTT
1051 GGGGGCATCA CGGTGCTGAC CAGGGGTGAC AGCACTTCCA GTACTAGGAG
1101 TACCGGGAAG CTGTCCAAAG CAGGTTCCGA GTCTTCCAGC AGTGCAGGCT
1151 CCTCAGGATC GCTGTCCCGC ACCCATCCAC CTCTCCAGAG CACACCCCTA
1201 GTCTCAGGTG TGGCAGCTGG CTCTCCAGGC TGTGTGCCTT ATCCAGAGAA
1251 TGGAATAGGG GGCCAGGTTG CTCCCAGCAG CACCAGCTAC ATCCTCCTTC
1301 CACTTGAAGC TGCAACAGGC ATCCCGCCTG GAAGCATCCT TCTTAATCCA
1351 CACACAGGCC AGCCCTTTGT GAATCCCGAT GGAACTCCTG CAATATACAA
1401 CCCACCCACC AGTCAGCAGC CCCTGCGAAG CGCCATGGTG GGGCAGTCCC
1451 AACAGCAGCC GCCACAGCAG CAGCCCTCCC CGCAGCCCCA ACAGCAGGTC
1501 CAGCCACCGC AGCCACAGAT GGCAGGCCCT CTGGTCACTC AGTCTGTCCA
1551 GGGGCTGCAG GCTTCCTCCC AGTCAGTGCA ATATCCGGCA GTCTCTTTTC
1601 CTCCCCAGCA CCTCCTACCT GTGTCTCCAA CGCAGCACTT TCCCATGAGA
1651 GATGATGTGG CAACACAGTT TGGCCAGATG ACCCTGAGCC GGCAGTCCTC
1701 GGGGGAGACT CCTGAACCCC CATCAGGTCC TGTCTACCCA TCCTCCCTTA
1751 TGCCACAGCC GGCCCAGCAG CCCAGCTATG TAATCGCCTC TACAGGCCAG
1801 CAGCTTCCTA CAGGAGGATT CTCAGGCTCT GGCCCTCCCA TCTCCCAGCA
1851 GGTCCTCCAG CCCCCTCCCT CACCACAGGG ATTCGTGCAA CAGCCTCCGC
1901 CTGCACAGAT GCCTGTATAT TATTACCCAT CTGGTCAGTA CCCTACCTCA
1951 ACCACGCAAC AGTACCGGCC CATGGCCCCG GTTCAGTACA ACGCTCAGAG
2001 GAGTCAACAG ATGCCACAGG CAGCACAGCA AGCAGGTTAC CAGCCAGTCT
2051 TGTCTGGTCA ACAGGGATTC CAAGGCCTAA TAGGAGTGCA GCAGCCACCT
2101 CAGAGTCAGA ACGTGATAAA TAACCAACAA GGAACTCCGG TGCAAAGCGT
2151 GATGGTTTCC TACCCAACAA TGTCTTCTTA TCAGGTGCCA ATGACCCAGG
2201 GTTCTCAAGG ACTGCCCCAG CAGTCATACC AACAGCCAAT CATGCTACCT
2251 AACCAGGCAG GTCAAGGGTC ACTCCCAGCC ACTGGAATGC CTGTTTACTG
2301 TAATGTCACA CCGCCCACCC CTCAGAACAA CCTTAGGCTG ATTGGCCCAC
2351 ACTGCCCCTC CAGCACTGTC CCAGTGATGT CAGCTAGCTG CAGAACAAAC
2401 TGTGCAAGTA TGAGCAATGC TGGTTGGCAG GTCAAATTCT GA
```

# Fig. 7: SEQ ID NO. 3: nucleotide sequence of human TARPP cDNA

**Length: 3212 bp**

```
   1  GTGATTTGCT GGAAGCTGGT CATTAGTGTT GACGATGTGT CACACTGTGT
  51  AAGGGAATCG CATGGAGATG GGCATTCCGA ACTGTTAATG GGGACATGGG
 101  ACTCCAGTTG TCTCTGATCA CTTGTGTGGA TTTTCCTGGC GTAGAACGAC
 151  AGAAGCCGCT AGTAAGTCGC CAAGACCTAC AGCAGGAATT CTGCACCAAA
 201  GGGCATAAAA TCTTGTTATT TTAATTTGCA TCTGGGAGAA TGTCTGAGCA
 251  AGGAGACCTG AATCAGGCAA TAGCAGAGGA AGGAGGGACT GAGCAGGAGA
 301  CGGCCACTCC AGAGAACGGC ATTGTTAAAT CAGAAAGTCT GGATGAAGAG
 351  GAGAAACTGG AACTGCAGAG GCGGCTGGAG GCTCAGAATC AAGAAAGAAG
 401  AAAATCCAAG TCAGGAGCAG GAAAAGGTAA ACTGACTCGC AGYCTTGCTG
 451  TCTGTGAGGA ATCTTCTGCC AGACCAGGAG GTGAAAGTCT TCAGGATCAG
 501  GAATCAATTC ATTTACAGCT TTCCAGTTTT TCCAGCCTGC AAGAGGAGGA
 551  TAAATCTAGG AAAGATGACT CTGAAAGAGA AAAAGAAAAG GATAAAAACA
 601  AAGATAAAAC CTCTGAAAAA CCCAAGATCA GAATGTTATC AAAAGATTGC
 651  AGCCAAGAAT ACACGGATTC TACAGGCATA GACTTACACG AGTTTCTGAT
 701  TAACACATTA AAGAATAATT CCAGGGACAG GATGATACTT TTGAAAATGG
 751  AGCAGGAAAT TATTGATTTC ATTGCTGACA ACAATAATCA TTATAAAAAG
 801  TTCCCTCAGA TGTCATCGTA TCAGAGGATG CTTGTCCATC GAGTGGCAGC
 851  TTATTTTGGA TTGGATCACA ATGTGGATCA AACAGGAAAA TCTGTTATCA
 901  TCAACAAGAC CAGCAGCACC AGAATACCAG AGCAAAGGTT TTGTGAACAT
 951  TTAAAAGATG AAAAAGGTGA AGAATCCCAG AAGCGGTTTA TCTTGAAGCG
1001  AGATAACTCT AGTATTGATA AAGAAGACAA TCAGTCAGTT TGCTCCCAGG
1051  AAAGCCTTTT TGTGGAAAAC AGTAGGCTCT TGGAAGACAG TAACATATGC
1101  AATGAGACCT ATAAGAAAAG ACAGCTCTTT CGGGGCAACA GAGATGGCTC
1151  AGGGAGAACA TCTGGGAGTC GACAGAGCAG CTCAGAAAAT GAACTCAAGT
1201  GGTCTGACCA CCAAAGGGCC TGGAGCAGCA CAGACTCCGA CAGTTCCAAC
1251  CGCAATCTAA AGCCCGCCAT GACCAAGACG GCGAGTTTTG GGGGCATCAC
1301  GGTGCTGACC AGGGGTGACA GCACTTCCAG TACTAGGAGT ACCGGGAAGC
1351  TGTCCAAAGC AGGTTCCGAG TCTTCCAGCA GTGCAGGCTC CTCAGGATCG
1401  CTGTCCCGCA CCCATCCACC TCTCCAGAGC ACACCCCTAG TCTCAGGTGT
1451  GGCAGCTGGC TCTCCAGGCT GTGTGCCTTA TCCAGAGAAT GGAATAGGGG
1501  GCCAGGTTGC TCCCAGCAGC ACCAGCTACA TCCTCCTTCC ACTTGAAGCT
1551  GCAACAGGCA TCCCGCCTGG AAGCATCCTT CTTAATCCAC ACACAGGCCA
1601  GCCCTTTGTG AATCCCGATG GAACTCCTGC AATATACAAC CCACCCACCA
1651  GTCAGCAGCC CCTGCGAAGC GCCATGGTGG GGCAGTCCCA ACAGCAGCCG
1701  CCACAGCAGC AGCCCTCCCC GCAGCCCCAA CAGCAGGTCC AGCCACCGCA
1751  GCCACAGATG GCAGGCCCTC TGGTCACTCA GTCTGTCCAG GGGCTGCAGG
1801  CTTCCTCCCA GTCAGTGCAA TATCCGGCAG TCTCTTTTCC TCCCCAGCAC
1851  CTCCTACCTG TGTCTCCAAC GCAGCACTTT CCCATGAGAG ATGATGTGGC
1901  AACACAGTTT GGCCAGATGA CCCTGAGCCG GCAGTCCTCG GGGGAGACTC
1951  CTGAACCCCC ATCAGGTCCT GTCTACCCAT CCTCCCTTAT GCCACAGCCG
2001  GCCCAGCAGC CCAGCTATGT AATCGCCTCT ACAGGCCAGC AGCTTCCTAC
2051  AGGAGGATTC TCAGGCTCTG GCCCTCCCAT CTCCCAGCAG GTCCTCCAGC
2101  CCCCTCCCTC ACCACAGGGA TTCGTGCAAC AGCCTCCGCC TGCACAGATG
2151  CCTGTATATT ATTACCCATC TGGTCAGTAC CCTACCTCAA CCACGCAACA
2201  GTACCGGCCC ATGGCCCCGG TTCAGTACAA CGCTCAGAGG AGTCAACAGA
2251  TGCCACAGGC AGCACAGCAA GCAGGTTACC AGCCAGTCTT GTCTGGTCAA
2301  CAGGGATTCC AAGGCCTAAT AGGAGTGCAG CAGCCACCTC AGAGTCAGAA
```

```
2351  CGTGATAAAT AACCAACAAG GAACTCCGGT GCAAAGCGTG ATGGTTTCCT
2401  ACCCAACAAT GTCTTCTTAT CAGGTGCCAA TGACCCAGGG TTCTCAAGGA
2451  CTGCCCCAGC AGTCATACCA ACAGCCAATC ATGCTACCTA ACCAGGCAGG
2501  TCAAGGGTCA CTCCCAGCCA CTGGAATGCC TGTTTACTGT AATGTCACAC
2551  CGCCCACCCC TCAGAACAAC CTTAGGCTGA TTGGCCCACA CTGCCCCTCC
2601  AGCACTGTCC CAGTGATGTC AGCTAGCTGC AGAACAAACT GTGCAAGTAT
2651  GAGCAATGCT GGTTGGCAGG TCAAATTCTG AGAGCTCTGG CTGTGGTACA
2701  TTTCTTCAGA TATTTCTCAT GGCCTTTGAT GGAAGAGGAA CAAGGTGGGA
2751  AAACTGGCTG AGGACTTAAG TATTCACTCA ACACTCAAAT GATTGCTGCT
2801  GGTATTCTGT AAAAAGTAAA CAAAGACTAA TATACACGTT AGCTGGTTAA
2851  TGGTGCATAT TTCTGTCATG TCTGCTAGGT ATGCCTTTAT AGCTTAGCTA
2901  GTGACATGAA TTCATCAAGG TAAGATTCTC TCCTACCACT GAATACCACT
2951  GTGTAGATTA TAATATCCCT AATTTGGATT AGTTTTGTAC TTTGTGTTGA
3001  GTTTGTGATG CTAAAAGTAT TTAAAAATTA TATACTAAAT CACATTGTAC
3051  CAAAGCTGTA ATGGAAAAGC AAAGAAGAAC TGATGAATTG AAGGAATAAT
3101  TTATATACAT TATAGAGTTT TCTTTTTTAA TGGATATATA CTGTATTGTA
3151  GTGTTTAATC AAAATAAAAC TATTTGACCT TATGGAGGAA GGTCATGTTT
3201  TTACCACTAA AA
```

# Fig. 8: SEQ ID NO. 4

**Length: 69 bp**

2471 ACAGCCAATCATGCTACCTAACCAGGCAGGTCAAGGGTCACTCCCAGCCA
CTGGAATGCCTGTTTACTG 2539

**Fig. 9: Schematic alignment of SEQ ID NO. 3, SEQ ID NO. 2 and SEQ ID NO. 4 with Genome Database EST-cluster**

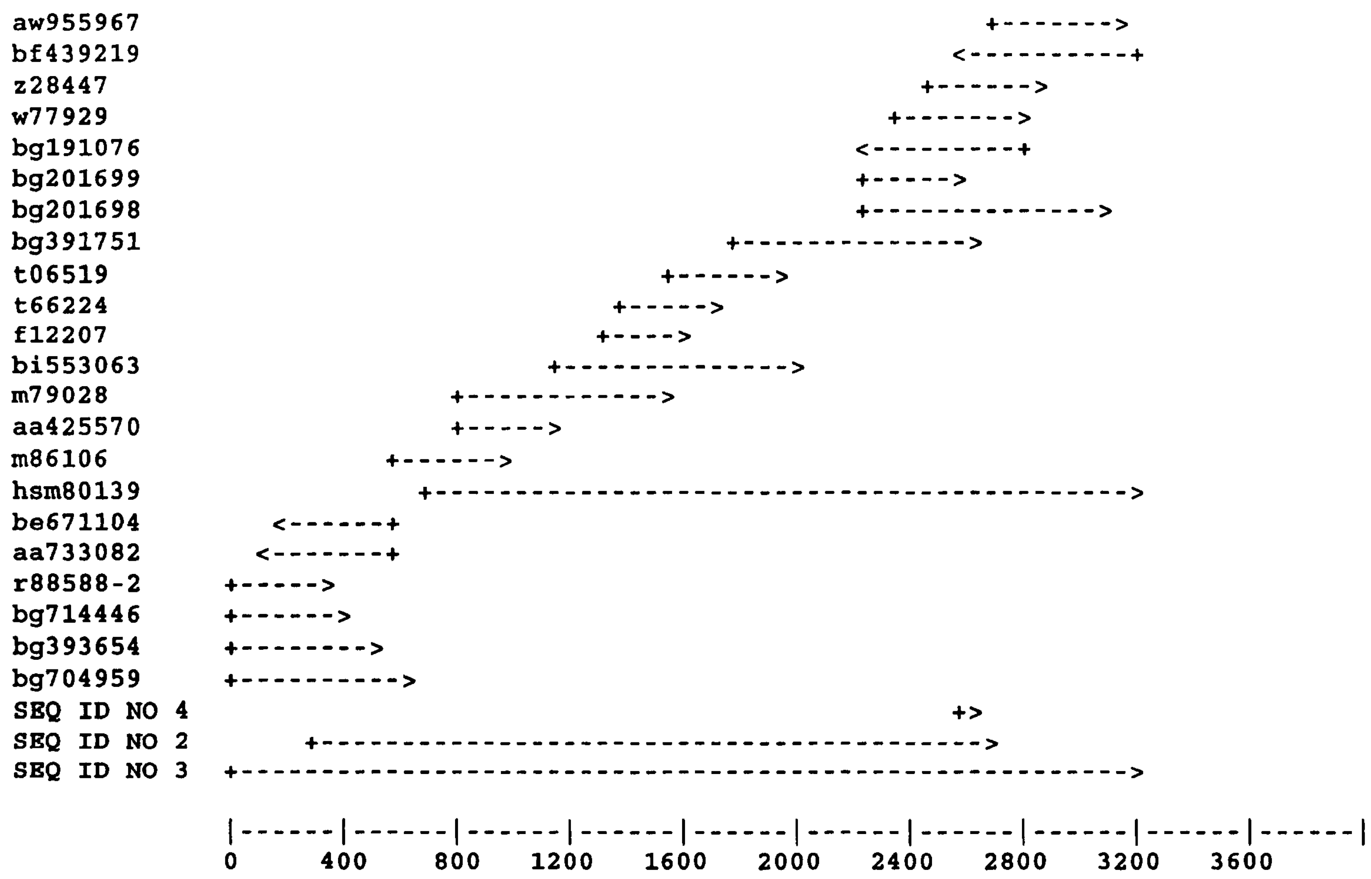

**Fig. 10: Images of the human cerebral cortex labeled with anti-hTARPP antiserumand withDAPI**

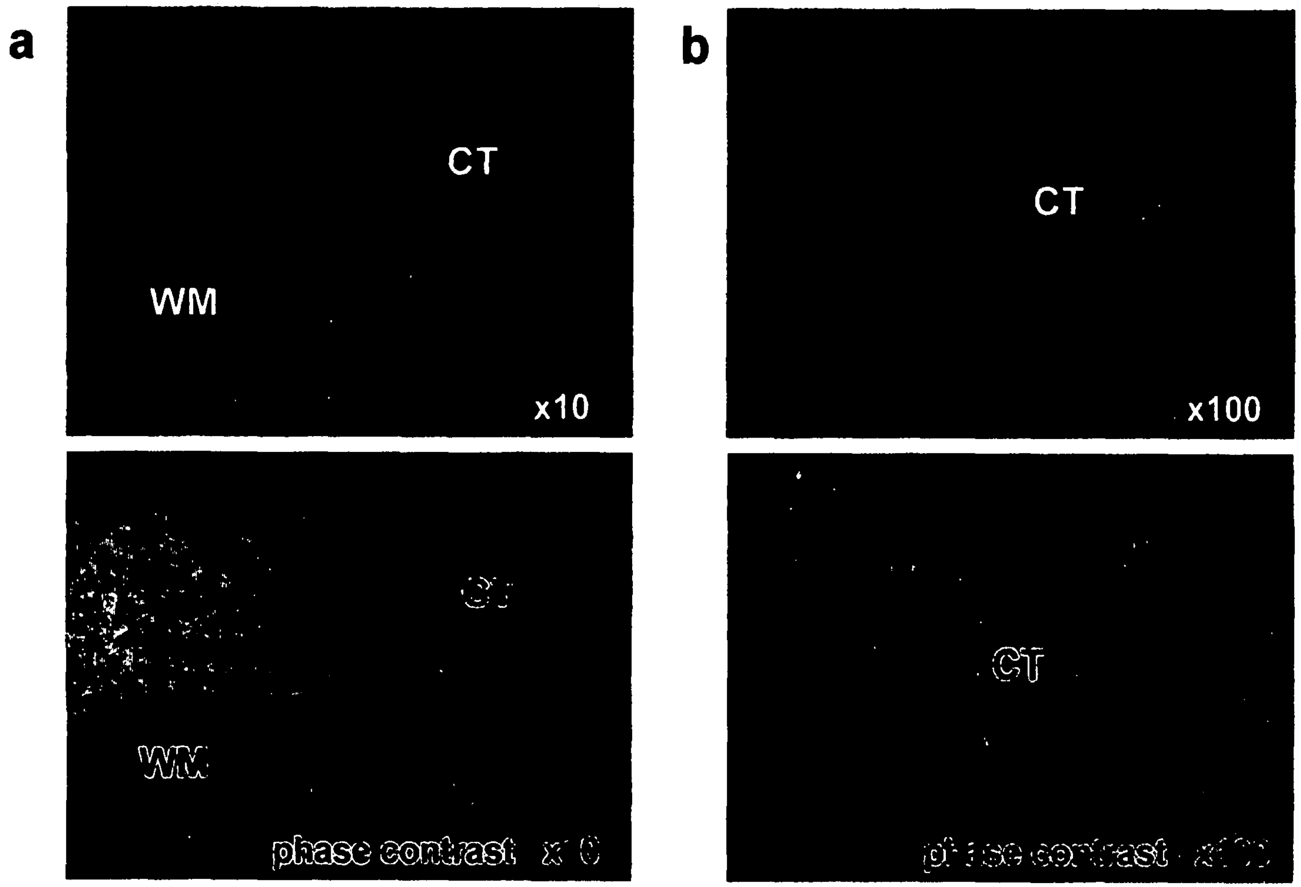

**Table 1:**

| sample | | Δ (fold) (frontal / temporal cortex) |
|---|---|---|
| patient | P012 | 2.21 |
| patient | P016 | 1.90 |
| patient | P010 | 4.11 |
| patient | P011 | 2.30 |
| patient | P014 | 1.73 |
| patient | P017 | 1.34 |
| patient | P019 | 2.62 |
| control | C011 | 1.24 |
| control | C012 | 1.15 |
| control | C014 | 0.47 |
| control | C005 | 1.39 |
| control | C008 | 1.38 |

**Table 2:**

| sample | Δ (fold) (frontal cortex / hippocampus) |
|---|---|
| patient P012 | 1.50 |
| patient P016 | 3.10 |
| patient P010 | 5.51 |
| patient P011 | 2.36 |
| patient P014 | 1.21 |
| patient P019 | 1.43 |
| control C005 | 1.38 |
| control C008 | 1.10 |
| control C004 | 1.76 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0034477 A
- WO 9920273 A
- US 5777195 A
- US 6150173 A
- WO 0214543 A
- WO 0052451 A
- WO 0201226 A
- WO 9613744 A
- WO 9816814 A
- WO 9823942 A
- WO 9917086 A
- WO 9934195 A
- WO 0066985 A
- WO 0159436 A
- WO 0159416 A
- EP 02007522 A


### Non-patent literature cited in the description


- **Vickers et al.** *Progress in Neurobiology,* 2000, vol. 60, 139-165
- **Selkoe.** *Physiological Rev,* 2001, vol. 81, 741-66
- **Greenfield et al.** *Frontiers Bioscience,* 2000, vol. 5, D72-83
- **Braak ; Braak.** *Acta Neuropathol,* 1991, vol. 82, 239-259
- **Johnson ; Jenkins.** *J Alzheimers Dis,* 1996, vol. 1, 38-58
- **Johnson ; Hartigan.** *J Alzheimers Dis,* 1999, vol. 1, 329-351
- **Schmitt et al.** *Neurology,* 2000, vol. 55, 370-376
- **Terry et al.** *Annals of Neurology,* 1981, vol. 10, 184-92
- **Strittmatter et al.** *Proc Natl Acad Sci USA,* 1993, vol. 90, 1977-81
- **Roses.** *Ann NY Acad Sci,* 1998, vol. 855, 738-43
- **Myers et al.** *Science,* 2000, vol. 290, 2304-5
- **Bertram et al.** *Science,* 2000, vol. 290, 2303
- **Scott et al.** *Am J Hum Genet,* 2000, vol. 66, 922-32
- **Greengard.** *Science,* 2001, vol. 294, 1024-1030
- **Brene et al.** *J Neurosci,* 1994, vol. 14, 985-998
- **Ouimet et al.** *J Neurosci,* 1989, vol. 9, 865-875
- **Hemmings ; Greengard.** *J Neurosci,* 1989, vol. 9, 851-864
- **Tsou et al.** *J Neurochem,* 1993, vol. 60, 1043-1046
- **Caporaso et al.** *Neuropharmacology,* 2000, vol. 39, 1637-1644
- **Kim et al.** *J Neural Transm Suppl,* 2001, vol. 61, 263-272
- **Kisielow et al.** *Eur J Immunol,* 2001, vol. 31, 1141-1149
- Alzheimer's Disease and Related Disorders. **Iqbal ; Swaab ; Winblad ; Wisniewski.** Etiology, Pathogenesis and Therapeutics. Wiley & Sons, 1999
- **Scinto ; Daffner.** Early Diagnosis of Alzheimer's Disease. Humana Press, 2000
- **Mayeux ; Christen.** Epidemiology of Alzheimer's Disease: From Gene to Prevention. Springer Press, 1999
- **Younkin ; Tanzi ; Christen.** Presenilins and Alzheimer's Disease. Springer Press, 1998
- **Sambrook ; Russell.** Molecular Cloning: A Laboratory Manual. Cold Spring Harbor Laboratory Press, 2000
- **Harlow et al.** Antibodies, A Laboratory Manual. Cold Spring Harbor Laboratory Press, 1988
- **Dubel ; Breitling.** Recombinant Antibodies. Wiley-Liss, 1999
- **Harlow ; Lane.** Using Antibodies: A Laboratory Manual. Cold Spring Harbor Laboratory Press, 1999
- **Edwards R.** Immunodiagnostics: A Practical Approach. Oxford University Press, 1999
- **Sambrook ; Russell.** Molecular Cloning: A Laboratory Manual. Cold Spring Harbor Laboratory Press, 2001
- **Schena M.** Microarray Biochip Technology. Eaton Publishing, 2000
- **Edwards R.** Immunodiagnostics: A Practical Approach. Oxford University Press, 1999
- **Behr.** *Acc Chem Res,* 1993, vol. 26, 274-278
- **Mulligan.** *Science,* 1993, vol. 260, 926-931

- **Wolff.** *Curr Opin Neurobiol,* 1993, vol. 3, 743-748
- **Gillespie.** *DN&P,* 1992, vol. 5, 389-395
- **Agrawal ; Akhtar.** *Trends Biotechnol,* 1995, vol. 13, 197-199
- **Crooke.** *Biotechnology,* 1992, vol. 10, 882-6
- **Barinaga.** *Science,* 1993, vol. 262, 1512-1514
- **Wickstrom.** *Trends Biotechnol,* 1992, vol. 10, 281-287
- **Hannon.** *Nature,* 2002, vol. 418, 244-251
- **Mc Celland ; Pardee.** Expression Genetics: Accelerated and High-Throughput Methods. Eaton Publishing, 1999
- **Lanza et al.** *Nature Medicine,* 1999, vol. 9, 975-977
- **Peterson DA.** *Curr. Opin. Pharmacol.,* 2002, vol. 2, 34-42
- **Colman A.** *Drug Discovery World,* 2001, vol. 7, 66-71
- **Capecchi.** *Science,* 1989, vol. 244, 1288-1292
- **Hogan et al.** Manipulating the Mouse Embryo: A Laboratory Manual. Cold Spring Harbor Laboratory Press, 1994
- **Jackson ; Abbott.** Mouse Genetics and Transgenics: A Practical Approach. Oxford University Press, 1999
- **Terry et al.** *Annals of Neurology,* 1981, vol. 10, 184-192
- Brain Biochemistry and Brain Disorders. Oxford University Press, 1992, 4

SEQUENCE LISTING

<110> Evotec NeuroSciences GmbH
<120> cAMP-Regulated Phosphorprotein for Diagnostic and Therapeutic Use in Neurodegenerative Diseases
<130> 020880ep
<140> EP02007522.2
<141> 2002-04-02
<160> 16
<170> PatentIn Ver. 2.1
<210> 1
<211> 813
<212> PRT
<213> Homo sapiens
<400> 1

```
Met Ser Glu Gln Gly Asp Leu Asn Gln Ala Ile Ala Glu Glu Gly Gly
  1               5                  10                  15

Thr Glu Gln Glu Thr Ala Thr Pro Glu Asn Gly Ile Val Lys Ser Glu
             20                  25                  30

Ser Leu Asp Glu Glu Glu Lys Leu Glu Leu Gln Arg Arg Leu Glu Ala
         35                  40                  45

Gln Asn Gln Glu Arg Arg Lys Ser Lys Ser Gly Ala Gly Lys Gly Lys
     50                  55                  60

Leu Thr Arg Ser Leu Ala Val Cys Glu Glu Ser Ser Ala Arg Pro Gly
 65                  70                  75                  80

Gly Glu Ser Leu Gln Asp Gln Glu Ser Ile His Leu Gln Leu Ser Ser
                 85                  90                  95

Phe Ser Ser Leu Gln Glu Glu Asp Lys Ser Arg Lys Asp Asp Ser Glu
            100                 105                 110

Arg Glu Lys Glu Lys Asp Lys Asn Lys Asp Lys Thr Ser Glu Lys Pro
        115                 120                 125

Lys Ile Arg Met Leu Ser Lys Asp Cys Ser Gln Glu Tyr Thr Asp Ser
    130                 135                 140
```

```
Thr Gly Ile Asp Leu His Glu Phe Leu Ile Asn Thr Leu Lys Asn Asn
145                 150                 155                 160

Ser Arg Asp Arg Met Ile Leu Leu Lys Met Glu Gln Glu Ile Ile Asp
                165                 170                 175

Phe Ile Ala Asp Asn Asn Asn His Tyr Lys Lys Phe Pro Gln Met Ser
            180                 185                 190

Ser Tyr Gln Arg Met Leu Val His Arg Val Ala Ala Tyr Phe Gly Leu
        195                 200                 205

Asp His Asn Val Asp Gln Thr Gly Lys Ser Val Ile Ile Asn Lys Thr
    210                 215                 220

Ser Ser Thr Arg Ile Pro Glu Gln Arg Phe Cys Glu His Leu Lys Asp
225                 230                 235                 240

Glu Lys Gly Glu Glu Ser Gln Lys Arg Phe Ile Leu Lys Arg Asp Asn
                245                 250                 255

Ser Ser Ile Asp Lys Glu Asp Asn Gln Ser Val Cys Ser Gln Glu Ser
            260                 265                 270

Leu Phe Val Glu Asn Ser Arg Leu Leu Glu Asp Ser Asn Ile Cys Asn
        275                 280                 285

Glu Thr Tyr Lys Lys Arg Gln Leu Phe Arg Gly Asn Arg Asp Gly Ser
    290                 295                 300

Gly Arg Thr Ser Gly Ser Arg Gln Ser Ser Ser Glu Asn Glu Leu Lys
305                 310                 315                 320

Trp Ser Asp His Gln Arg Ala Trp Ser Ser Thr Asp Ser Asp Ser Ser
                325                 330                 335

Asn Arg Asn Leu Lys Pro Ala Met Thr Lys Thr Ala Ser Phe Gly Gly
            340                 345                 350

Ile Thr Val Leu Thr Arg Gly Asp Ser Thr Ser Ser Thr Arg Ser Thr
        355                 360                 365

Gly Lys Leu Ser Lys Ala Gly Ser Glu Ser Ser Ser Ser Ala Gly Ser
    370                 375                 380

Ser Gly Ser Leu Ser Arg Thr His Pro Pro Leu Gln Ser Thr Pro Leu
385                 390                 395                 400
```

```
Val Ser Gly Val Ala Ala Gly Ser Pro Gly Cys Val Pro Tyr Pro Glu
                405                 410                 415

Asn Gly Ile Gly Gly Gln Val Ala Pro Ser Ser Thr Ser Tyr Ile Leu
            420                 425                 430

Leu Pro Leu Glu Ala Ala Thr Gly Ile Pro Pro Gly Ser Ile Leu Leu
        435                 440                 445

Asn Pro His Thr Gly Gln Pro Phe Val Asn Pro Asp Gly Thr Pro Ala
    450                 455                 460

Ile Tyr Asn Pro Pro Thr Ser Gln Gln Pro Leu Arg Ser Ala Met Val
465                 470                 475                 480

Gly Gln Ser Gln Gln Gln Pro Pro Gln Gln Gln Pro Ser Pro Gln Pro
                485                 490                 495

Gln Gln Gln Val Gln Pro Pro Gln Pro Gln Met Ala Gly Pro Leu Val
            500                 505                 510

Thr Gln Ser Val Gln Gly Leu Gln Ala Ser Ser Gln Ser Val Gln Tyr
        515                 520                 525

Pro Ala Val Ser Phe Pro Pro Gln His Leu Leu Pro Val Ser Pro Thr
    530                 535                 540

Gln His Phe Pro Met Arg Asp Asp Val Ala Thr Gln Phe Gly Gln Met
545                 550                 555                 560

Thr Leu Ser Arg Gln Ser Ser Gly Glu Thr Pro Glu Pro Pro Ser Gly
                565                 570                 575

Pro Val Tyr Pro Ser Ser Leu Met Pro Gln Pro Ala Gln Gln Pro Ser
            580                 585                 590

Tyr Val Ile Ala Ser Thr Gly Gln Gln Leu Pro Thr Gly Gly Phe Ser
        595                 600                 605

Gly Ser Gly Pro Pro Ile Ser Gln Gln Val Leu Gln Pro Pro Pro Ser
    610                 615                 620

Pro Gln Gly Phe Val Gln Gln Pro Pro Pro Ala Gln Met Pro Val Tyr
625                 630                 635                 640

Tyr Tyr Pro Ser Gly Gln Tyr Pro Thr Ser Thr Thr Gln Gln Tyr Arg
                645                 650                 655
```

```
Pro Met Ala Pro Val Gln Tyr Asn Ala Gln Arg Ser Gln Gln Met Pro
            660                 665                 670

Gln Ala Ala Gln Gln Ala Gly Tyr Gln Pro Val Leu Ser Gly Gln Gln
        675                 680                 685

Gly Phe Gln Gly Leu Ile Gly Val Gln Gln Pro Pro Gln Ser Gln Asn
    690                 695                 700

Val Ile Asn Asn Gln Gln Gly Thr Pro Val Gln Ser Val Met Val Ser
705                 710                 715                 720

Tyr Pro Thr Met Ser Ser Tyr Gln Val Pro Met Thr Gln Gly Ser Gln
                725                 730                 735

Gly Leu Pro Gln Gln Ser Tyr Gln Gln Pro Ile Met Leu Pro Asn Gln
            740                 745                 750

Ala Gly Gln Gly Ser Leu Pro Ala Thr Gly Met Pro Val Tyr Cys Asn
        755                 760                 765

Val Thr Pro Pro Thr Pro Gln Asn Asn Leu Arg Leu Ile Gly Pro His
    770                 775                 780

Cys Pro Ser Ser Thr Val Pro Val Met Ser Ala Ser Cys Arg Thr Asn
785                 790                 795                 800

Cys Ala Ser Met Ser Asn Ala Gly Trp Gln Val Lys Phe
                805                 810
```

<210> 2
<211> 2442
<212> DNA
<213> Homo sapiens
<400> 2

```
atgtctgagc aaggagacct gaatcaggca atagcagagg aaggagggac tgagcaggag 60
acggccactc cagagaacgg cattgttaaa tcagaaagtc tggatgaaga ggagaaactg 120
gaactgcaga ggcggctgga ggctcagaat caagaaagaa gaaaatccaa gtcaggagca 180
ggaaaaggta aactgactcg cagycttgct gtctgtgagg aatcttctgc cagaccagga 240
ggtgaaagtc ttcaggatca ggaatcaatt catttacagc tttccagttt ttccagcctg 300
caagaggagg ataaatctag gaaagatgac tctgaaagag aaaaagaaaa ggataaaaac 360
aaagataaaa cctctgaaaa acccaagatc agaatgttat caaaagattg cagccaagaa 420
tacacggatt ctacaggcat agacttacac gagtttctga ttaacacatt aaagaataat 480
tccagggaca ggatgatact tttgaaaatg gagcaggaaa ttattgattt cattgctgac 540

aacaataatc attataaaaa gttccctcag atgtcatcgt atcagaggat gcttgtccat 600
cgagtggcag cttattttgg attggatcac aatgtggatc aaacaggaaa atctgttatc 660
atcaacaaga ccagcagcac cagaatacca gagcaaaggt tttgtgaaca tttaaaagat 720
gaaaaaggtg aagaatccca gaagcggttt atcttgaagc gagataactc tagtattgat 780
aaagaagaca atcagtcagt ttgctcccag gaaagccttt ttgtggaaaa cagtaggctc 840
ttggaagaca gtaacatatg caatgagacc tataagaaaa gacagctctt tcggggcaac 900
agagatggct cagggagaac atctgggagt cgacagagca gctcagaaaa tgaactcaag 960
tggtctgacc accaaagggc ctggagcagc acagactccg acagttccaa ccgcaatcta 1020
aagcccgcca tgaccaagac ggcgagtttt gggggcatca cggtgctgac caggggtgac 1080
agcacttcca gtactaggag taccgggaag ctgtccaaag caggttccga gtcttccagc 1140
agtgcaggct cctcaggatc gctgtcccgc acccatccac ctctccagag cacaccccta 1200
gtctcaggtg tggcagctgg ctctccaggc tgtgtgcctt atccagagaa tggaataggg 1260
ggccaggttg ctcccagcag caccagctac atcctccttc cacttgaagc tgcaacaggc 1320
atcccgcctg gaagcatcct tcttaatcca cacacaggcc agccctttgt gaatcccgat 1380
ggaactcctg caatatacaa cccacccacc agtcagcagc ccctgcgaag cgccatggtg 1440
gggcagtccc aacagcagcc gccacagcag cagccctccc cgcagcccca acagcaggtc 1500
cagccaccgc agccacagat ggcaggccct ctggtcactc agtctgtcca ggggctgcag 1560
gcttcctccc agtcagtgca atatccggca gtctcttttc ctccccagca cctcctacct 1620
gtgtctccaa cgcagcactt tcccatgaga gatgatgtgg caacacagtt tggccagatg 1680
accctgagcc ggcagtcctc gggggagact cctgaacccc catcaggtcc tgtctaccca 1740
tcctccctta tgccacagcc ggcccagcag cccagctatg taatcgcctc tacaggccag 1800
cagcttccta caggaggatt ctcaggctct ggccctccca tctcccagca ggtcctccag 1860
ccccctccct caccacaggg attcgtgcaa cagcctccgc ctgcacagat gcctgtatat 1920
tattacccat ctggtcagta ccctacctca accacgcaac agtaccggcc catggccccg 1980
gttcagtaca acgctcagag gagtcaacag atgccacagg cagcacagca agcaggttac 2040
cagccagtct tgtctggtca acagggattc caaggcctaa taggagtgca gcagccacct 2100
cagagtcaga acgtgataaa taaccaacaa ggaactccgg tgcaaagcgt gatggtttcc 2160
tacccaacaa tgtcttctta tcaggtgcca atgacccagg gttctcaagg actgccccag 2220
cagtcatacc aacagccaat catgctacct aaccaggcag gtcaagggtc actcccagcc 2280
actggaatgc ctgtttactg taatgtcaca ccgcccaccc ctcagaacaa ccttaggctg 2340
attggcccac actgcccctc cagcactgtc ccagtgatgt cagctagctg cagaacaaac 2400
tgtgcaagta tgagcaatgc tggttggcag gtcaaattct ga                   2442
```

<210> 3
<211> 3212

<212> DNA
<213> Homo sapiens
<400> 3

```
gtgatttgct ggaagctggt cattagtgtt gacgatgtgt cacactgtgt aagggaatcg 60
catggagatg ggcattccga actgttaatg gggacatggg actccagttg tctctgatca 120
cttgtgtgga ttttcctggc gtagaacgac agaagccgct agtaagtcgc caagacctac 180
agcaggaatt ctgcaccaaa gggcataaaa tcttgttatt ttaatttgca tctgggagaa 240
tgtctgagca aggagacctg aatcaggcaa tagcagagga aggagggact gagcaggaga 300
cggccactcc agagaacggc attgttaaat cagaaagtct ggatgaagag gagaaactgg 360
aactgcagag gcggctggag gctcagaatc aagaaagaag aaaatccaag tcaggagcag 420
gaaaaggtaa actgactcgc agycttgctg tctgtgagga atcttctgcc agaccaggag 480
```

```
gtgaaagtct tcaggatcag gaatcaattc atttacagct ttccagtttt tccagcctgc 540
aagaggagga taaatctagg aaagatgact ctgaaagaga aaaagaaaag gataaaaaca 600
aagataaaac ctctgaaaaa cccaagatca gaatgttatc aaaagattgc agccaagaat 660
acacggattc tacaggcata gacttacacg agtttctgat taacacatta aagaataatt 720
ccagggacag gatgatactt ttgaaaatgg agcaggaaat tattgatttc attgctgaca 780
acaataatca ttataaaaag ttccctcaga tgtcatcgta tcagaggatg cttgtccatc 840
gagtggcagc ttattttgga ttggatcaca atgtggatca aacaggaaaa tctgttatca 900
tcaacaagac cagcagcacc agaataccag agcaaaggtt ttgtgaacat ttaaaagatg 960
aaaaaggtga agaatcccag aagcggttta tcttgaagcg agataactct agtattgata 1020
aagaagacaa tcagtcagtt tgctcccagg aaagcctttt tgtggaaaac agtaggctct 1080
tggaagacag taacatatgc aatgagacct ataagaaaag acagctcttt cggggcaaca 1140
gagatggctc agggagaaca tctgggagtc gacagagcag ctcagaaaat gaactcaagt 1200
ggtctgacca ccaaagggcc tggagcagca cagactccga cagttccaac cgcaatctaa 1260
agcccgccat gaccaagacg gcgagttttg ggggcatcac ggtgctgacc aggggtgaca 1320
gcacttccag tactaggagt accgggaagc tgtccaaagc aggttccgag tcttccagca 1380
gtgcaggctc ctcaggatcg ctgtcccgca cccatccacc tctccagagc acacccctag 1440
tctcaggtgt ggcagctggc tctccaggct gtgtgcctta tccagagaat ggaatagggg 1500
gccaggttgc tcccagcagc accagctaca tcctccttcc acttgaagct gcaacaggca 1560
tcccgcctgg aagcatcctt cttaatccac acacaggcca gccctttgtg aatcccgatg 1620
gaactcctgc aatatacaac ccacccacca gtcagcagcc cctgcgaagc gccatggtgg 1680
ggcagtccca acagcagccg ccacagcagc agccctcccc gcagccccaa cagcaggtcc 1740
agccaccgca gccacagatg gcaggccctc tggtcactca gtctgtccag ggggctgcagg 1800
cttcctccca gtcagtgcaa tatccggcag tctcttttcc tccccagcac ctcctacctg 1860
tgtctccaac gcagcacttt cccatgagag atgatgtggc aacacagttt ggccagatga 1920
ccctgagccg gcagtcctcg gggagactc ctgaaccccc atcaggtcct gtctacccat 1980
cctcccttat gccacagccg gcccagcagc ccagctatgt aatcgcctct acaggccagc 2040
agcttcctac aggaggattc tcaggctctg gccctcccat ctcccagcag gtcctccagc 2100
cccctccctc accacaggga ttcgtgcaac agcctccgcc tgcacagatg cctgtatatt 2160
attacccatc tggtcagtac cctacctcaa ccacgcaaca gtaccggccc atggccccgg 2220
ttcagtacaa cgctcagagg agtcaacaga tgccacaggc agcacagcaa gcaggttacc 2280
agccagtctt gtctggtcaa cagggattcc aaggcctaat aggagtgcag cagccacctc 2340
agagtcagaa cgtgataaat aaccaacaag gaactccggt gcaaagcgtg atggtttcct 2400
acccaacaat gtcttcttat caggtgccaa tgacccaggg ttctcaagga ctgccccagc 2460
agtcatacca acagccaatc atgctaccta accaggcagg tcaagggtca ctcccagcca 2520
ctggaatgcc tgtttactgt aatgtcacac cgcccacccc tcagaacaac cttaggctga 2580
ttggcccaca ctgcccctcc agcactgtcc cagtgatgtc agctagctgc agaacaaact 2640
gtgcaagtat gagcaatgct ggttggcagg tcaaattctg agagctctgg ctgtggtaca 2700
tttcttcaga tatttctcat ggcctttgat ggaagaggaa caaggtggga aaactggctg 2760
aggacttaag tattcactca acactcaaat gattgctgct ggtattctgt aaaaagtaaa 2820
caaagactaa tatacacgtt agctggttaa tggtgcatat ttctgtcatg tctgctaggt 2880
atgcctttat agcttagcta gtgacatgaa ttcatcaagg taagattctc tcctaccact 2940
gaataccact gtgtagatta taatatccct aatttggatt agttttgtac tttgtgttga 3000
gtttgtgatg ctaaaagtat ttaaaaatta tatactaaat cacattgtac caaagctgta 3060
atggaaaagc aaagaagaac tgatgaattg aaggaataat ttatatacat tatagagttt 3120
tcttttttaa tggatatata ctgtattgta gtgtttaatc aaaataaaac tatttgacct 3180
tatggaggaa ggtcatgttt ttaccactaa aa                               3212
```

<210> 4
<211> 69
<212> DNA
<213> Artificial Sequence
<220>
<223> Description of Artificial Sequence: PCR Amplified DNA
<400> 4

acagccaatc atgctaccta accaggcagg tcaagggtca ctcccagcca ctggaatgcc 60
tgtttactg 69

<210> 5
<211> 23
<212> DNA
<213> Artificial Sequence
<220>
<223> Description of Artificial Sequence: DNA Primer
<400> 5
acagccaatc atgctaccta acc 23
<210> 6
<211> 23
<212> DNA
<213> Artificial Sequence
<220>
<223> Description of Artificial Sequence: DNA Primer
<400> 6
acagtaaaca ggcattccag tgg 23
<210> 7
<211> 20
<212> DNA
<213> Artificial Sequence
<220>
<223> Description of Artificial Sequence: DNA Primer
<400> 7
actgaagcac tacgggcctg 20
<210> 8
<211> 19
<212> DNA
<213> Artificial Sequence
<220>
<223> Description of Artificial Sequence: DNA Primer
<400> 8
agccgttggt gtctttgcc 19
<210> 9
<211> 20
<212> DNA
<213> Artificial Sequence
<220>
<223> Description of Artificial Sequence: DNA Primer
<400> 9
ggtcaaattt accctggcca 20
<210> 10
<211> 22
<212> DNA
<213> Artificial Sequence
<220>
<223> Description of Artificial Sequence: DNA Primer

<400> 10
tctcatcaag cgtcagcagt tc 22
<210> 11
<211> 19
<212> DNA
<213> Artificial Sequence
<220>
<223> Description of Artificial Sequence: DNA Primer
<400> 11
tggaacggtg aaggtgaca 19
<210> 12
<211> 19
<212> DNA
<213> Artificial Sequence
<220>
<223> Description of Artificial Sequence: DNA Primer
<400> 12
ggcaagggac ttcctgtaa 19
<210> 13
<211> 20
<212> DNA
<213> Artificial Sequence
<220>
<223> Description of Artificial Sequence: DNA Primer
<400> 13
cgtcatgggt gtgaaccatg 20
<210> 14
<211> 21
<212> DNA
<213> Artificial Sequence
<220>
<223> Description of Artificial Sequence: DNA Primer
<400> 14
gctaagcagt tggtggtgca g 21
<210> 15
<211> 21
<212> DNA
<213> Artificial Sequence
<220>
<223> Description of Artificial Sequence: DNA Primer
<400> 15
gtcgctggtc agttcgtgat t 21
<210> 16
<211> 23
<212> DNA
<213> Artificial Sequence
<220>
<223> Description of Artificial Sequence: DNA Primer
<400> 16
agcagttggc tgttgtacct ctc 23